# EUROPEAN PATENT APPLICATION

(11) **EP 4 276 674 A1**
(43) Date of publication of application: **15.11.2023**
(21) Application number: 21911368.5
(22) Date of filing: 14.12.2021
(51) Int. Cl.: G06F 30/27, G01N 5/04, G01N 23/207, G01N 24/08, G01N 1/04, G01N 3/08, G01N 33/38, G01N 1/02, G01N 21/35

(54) **DEEP-LEARNING-BASED CONCRETE STRUCTURE DETERIORATION ASSESSMENT SYSTEM AND METHOD THEREFOR**

(30) Priority: 21.12.2020 KR 20200179209
(71) Applicant: Korea Institute Of Civil Engineering And Building Technology, Goyang-si, Gyeonggi-do 10223 (KR)
(72) Inventor: HEO, Young Sun, Hwaseong-si Gyeonggi-do 18266 (KR)
(74) Representative: Becker, Eberhard
(86) International application number: PCT/KR2021/018943
(87) International publication number: WO 2022/139301

(57) **Abstract**

Provided are a deep-learning-based concrete structure deterioration assessment system and a method therefor, the system: using a forensic investigation (FIS) analysis tool for concrete structure to assess, through deep-learning-based chemical analysis, most of the deterioration of a concrete structure, such as fire damage extent assessment, strength development assessment, carbonation damage extent assessment, salt damage extent assessment, and other chemical attack damage extent assessments; combining, during the application of a deep learning interface algorithm (DIA) of the FIS analysis tool for concrete structure, other field survey data in a big data storage unit, standardized experimental data, statistical data in history information and the like, and sample analysis results of the concrete structure obtained onsite from a specific location, so as to extend same at a sampling location and in member units or whole structure units and assess same; optimizing the assessed results to increase a prediction rate; and implementing physical and chemical correction for contamination sources of the concrete structure sample to increase the accuracy of the deterioration assessment results of the concrete structure, and clearly assessing the extent of deterioration damage of the concrete structure according to changes in depth to correctly diagnose the deterioration of concrete structure.

## Description

### [Technical Field]

The present invention relates to time-dependent assessment of concrete deterioration, and more particularly, to a deep learning-based system for time-dependent assessment of concrete deterioration and a method for the same that chemically analyze and assess, based on deep learning, most of concrete deteriorations, such as fire damage scale assessment, strength development assessment, carbonation damage scale assessment, salt damage scale assessment, and unclassified chemical attack damage scale assessment of a concrete structure from a concrete structure sample collected from the site.

### [Background Art]

In general, concrete may be regarded as a compound composed of cement paste and aggregate hardened according to a chemical reaction between cement and water. Here, the cement paste is composed of various types of hydration products, and each hydration product has a unique material property and affects concrete.

Among the hydration products of the cement paste, calcium silica hydrate (C-S-H) affects the development of strength of concrete, and calcium hydroxide (Ca(OH)₂) imparts alkalinity to concrete to prevent rebars in reinforced concrete structures from rusting.

However, when the key hydration products constituting such cement paste are chemically decomposed due to external environmental influences and lose their unique characteristics, the quality of concrete is directly affected. In this case, when appropriate measures are not collected, the deterioration of concrete continues, and in serious cases, the remaining life of concrete may be drastically shortened.

As the most representative external environments that cause such deterioration problems, there are a case where concrete is exposed to high temperatures such as fire, a case where concrete is exposed to extreme cold in winter, and a case where in which a significantly small amount of unit cement is used. These external environments can directly affect the strength of concrete.

In addition, these external environments may affect the remaining life of concrete in addition to the strength of concrete. Even in non-extreme situations, external harmful ions, such as carbon dioxide, may penetrate into concrete in a general atmospheric environment. For example, in coastal areas, chloride penetration into concrete is a representative cause of deterioration.

In addition, there are special substances that cause deterioration of concrete. Representative substances include corrosive gas, acid, salt, ad the like. Concrete deterioration caused by these external factors is called chemical attack, which may cause serious quality degradation and life reduction problems in concrete.

For the assessment of concrete deterioration, fire damage scale assessment, strength development assessment, carbonation damage scale assessment, salt damage scale assessment, unclassified chemical attack damage scale assessment, etc., may be performed.

Specifically, the fire damage scale assessment of concrete structures according to the related art is described as follows.

In general, when the concrete structure is exposed to high temperatures such as fire for a long time, a hardened cement and an aggregate each have different expansion and contraction behaviors, resulting in cracks or weakening of the structure due to chemical dissociation of the hydration products, As a result, the change in pore structure, physical properties, and the like may greatly deteriorate, huge cracks occur in concrete due to thermal stress caused by end restraints or the like, and in serious cases, the concrete may collapse.

Here, the hardened cement has a large amount of chemically-bonded water in addition to free water. When the cured product is exposed to 100 °C or higher, the free water present in capillary pores evaporates first, so the volume expands more than 1300 times. In addition, when the heating temperature rises to about 180 °C, some of the chemically-bonded water that is chemically bonded to the hardened cement starts to evaporate. At about 250 to 350 °C, about 20% of moisture contained in the calcium silicate hydration product, which is a key hydration product for the strength development of the hardened cement, is lost, and at 400 to 700 °C, most of the contained moisture is lost. In addition, in a similar temperature range, calcium hydroxide, a free alkali component in concrete, is thermally decomposed into quicklime and water, resulting in chemical damage and loss of rigidity due to the increase of rather large pores, which may become structurally very dangerous. Thereafter, when the concrete is heated at about 1200 °C or higher for a long time, the concrete gradually melts at the surface.

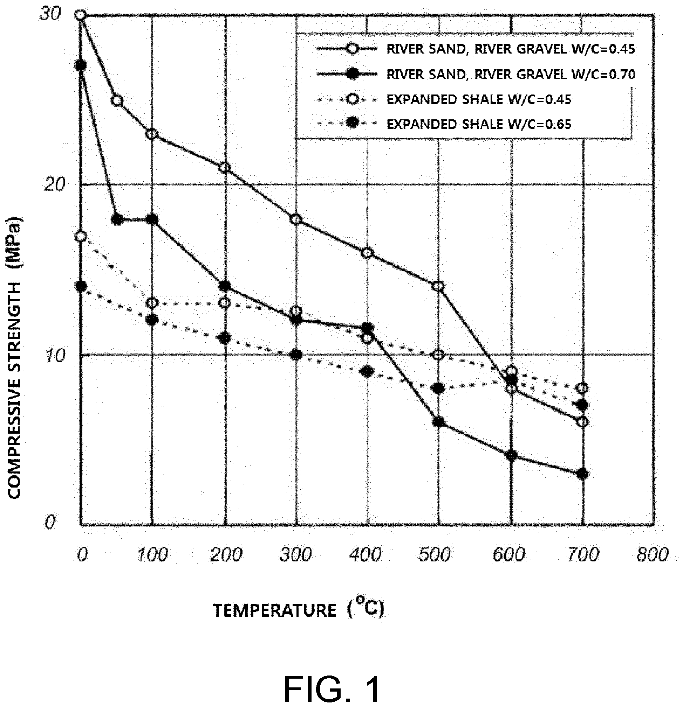

FIG. 1 is a diagram illustrating that a compressive strength of a concrete structure decreases as temperature rises during a fire.

As illustrated in FIG. 1, the concrete structure exhibits the opposite behavior in that the cement hydration product in the concrete is chemically decomposed as the heating temperature rises, and the cement paste shrinks but the aggregate expands as the concrete is exposed to high temperature. This is mainly because the internal destruction caused by a difference in thermal expansion coefficient according to the quality characteristics of the cement paste and the aggregate affects mechanical properties of concrete. In addition, as a result of moisture expansion of the free water and the like present in the concrete capillary pores, the internal stress gradually increases and the internal structure is destructed, resulting in a decrease in mechanical properties such as strength and elasticity.

In this case, the degree of deterioration varies depending on the type, blend, material age, and the like of the materials used, and shows a tendency as illustrated in FIG. 1. That is, there is almost no decrease in strength up to 300 °C, but the decrease in strength becomes less than 50% when it exceeds 500 °C. In addition, at about 700 °C, the compressive strength may decrease to about 60 to 80% of that at room temperature. Accordingly, it can be seen that the compressive strength greatly decreases when the concrete is subjected to high heat by a fire. In addition, it can be seen that the modulus of elasticity also decreases by heating and is almost halved at 500 °C. This is because, when the concrete is subjected to high heat, the concrete loses its elastic properties and gradually changes to plasticity.

In particular, the performance of the concrete structure decreases due to the change in the microstructure in the event of a fire. For example, when a gas adsorption method that measures the pore structure by adsorbing nitrogen at -195.8 °C, which is the boiling point of nitrogen, or porosimeter analysis by mercury intrusion is performed, such changes in the microstructure may be identified by pore distribution, pore structure characteristics, or the like.

Therefore, it is necessary to accurately diagnose the fire damage scale assessment of concrete structure having degraded concrete structure in order to determine whether to reuse the concrete structure having suffered fire damage and damage ratings of the concrete structure based on the fire mechanism in the concrete structure.

Meanwhile, the strength development assessment of the concrete structure according to the related art will be described as follows.

The strength of concrete is generally highly related to the amount of pores per unit volume and the homogeneity of the constituent materials. For example, mixing design, such as unit cement amount, aggregate granularity, curing temperature, curing humidity, curing period, and material separation, and quality control immediately after concrete is cast, are important.

Here, the chemical composition that most directly affects the strength of concrete is calcium silicate hydrate (C-S-H) gel, which is formed when cement and water meet. When cement substitutes are overused by unskilled technicians, the generation of such a C-S-H gel may be hindered, causing serious problems in early strength development.

In addition, as an environmental factor, when concrete is cast in sub-zero weather in winter, the hydration reaction itself does not occur as the water freezes, and as a result, the C-S-H gel does not exist or exists in a small amount.

Therefore, when there is any doubt as to whether the strength of the already cast concrete has developed at the site, countermeasures should be prepared through prompt diagnosis. For example, it is important to confirm whether hydration products are stably present in the cement paste inside on-site concrete. In particular, technologies capable of scientifically diagnosing the state of the C-S-H gel are needed.

For example, as the related art for checking whether the strength development of concrete already cast at the site, Korean Patent No. 10-983000 discloses an invention entitled "Temperature Follow-up Specimen Curing Device Capable of Displaying Integrated Temperature," and discloses that after a temperature sensor is embedded before on-site concrete casting, concrete is cast, temperature data measured in real time is recorded and an ambient temperature of a chamber in which the concrete specimen is placed in a laboratory condition is set to the same, when a certain period of time has elapsed, the specimen is taken out of the chamber and the strength is measured, and then, the strength of the specimen may be assessed in the same way as the strength of the structure at the site.

However, as the temperature follow-up specimen curing device capable of displaying the integrated temperature according to the related art can be applied only when there is a pre-inserted sensor and a specimen in a prepared laboratory chamber only for members where quality problems of concrete are expected, there is a problem in that it may not be applied to random locations where the post-quality is questionable after the construction structure is completed.

In addition, since the strength of the concrete structure is in a proportional relationship with the remaining life of concrete in addition to the first purpose of bearing the load of the structure itself, it is difficult to find an advanced technology that may scientifically assess the concrete structure after the concrete structure is completed at the construction site.

Meanwhile, the carbonation damage scale assessment of the concrete structure according to the related art will be described as follows.

In the cement hydration reaction, about 1/3 of the total hydration product is produced as calcium hydroxide (Ca(OH)₂). The calcium hydroxide produced in this way exists in a saturated aqueous solution state in crystals or concrete capillary pores, and shows strong alkalinity of about pH 12 to 13. The calcium hydroxide undergoes a chemical reaction due to the penetration of moisture and carbon dioxide in the air. As the reaction result, the calcium hydroxide is decomposed into calcium carbonate (CaCO₃) and water (H₂O), lowering the pH to 8.5 to 10, and thus, having neutralization property, which is called a carbonation (or neutralization) reaction.

Since the carbonation reaction itself is a contraction reaction, it does not affect the quality of concrete, but as the concrete changes from alkaline property to neutralization property, a thin passivation film formed in the alkaline environment on a surface of a rebar that was present inside is destructed. Then, when the rebar having the passivation film destructed meets water and oxygen penetrating from the outside, corrosion proceeds, and when no special measures are taken, the life span of the concrete is shortened due to corrosion expansion destruction.

For a method for identifying carbonation of concrete according to the related art, for example, powders dropped by coring (or drilling) may react with a phenophthalene solution to immediately check whether color has changed at the site.

However, in the case of the method of identifying carbonation of a phenophthalene solution, it is conducted for the purpose of a general safety diagnosis, and it is decided whether to carry out the reconstruction or observe over a longer period of time. In particular, it is possible to determine only how far carbonation has progressed by proceeding only when a concrete structure is already suspected of having serious life degradation. In addition, the method of identifying carbonation using such a phenophthalene solution is used in most sites, but if even some of the concrete is in the carbonation state, the carbonation reaction occurs, so there is a problem in that it is difficult to quantitatively assess the state of the entire member or the entire structure.

In other words, the method of identifying carbonation using a phenophthalene solution may roughly confirm only a carbonation depth of a specific location where the color of the phenophthalene solution has changed, and may not determine through the phenophthalene solution whether the carbonation has progressed 10% or 80% at the depth where the color change has occurred and may not confirm the location where the sample has not been collected at all, so there is a problem in that it is not possible to accurately predict the carbonation rate and the remaining life.

In addition, as other methods of assessing carbonation damage of a concrete structure according to the related art, there are X-ray diffraction, thermal analysis, C-S-H quantification, silica gel quantification, and the like. However, there is a problem in that these methods have difficult in analyzing only a cement matrix after removing an aggregate and an admixture from concrete, and thus, are difficult to actually apply to sites.

Meanwhile, the salt damage scale assessment of concrete structures according to the related art will be described in detail as follows.

In general, the presence of chloride in concrete is unavoidable, but if the amount of chloride is too excessive, steel materials buried for reinforcement are exposed to an environment where they are easily corroded.

For such chloride, a concentration ratio of Cl⁻/OH⁻ is important. In general, when the concentration ratio of Cl⁻/OH⁻ is managed to be 0.6 or lower, it is known that steel materials do not undergo corrosion. Compared to the total amount of calcium hydroxide present in concrete, it is known that the lower the amount of chloride, the better. For example, in general concrete, the limiting amount of chlorine ions in concrete is managed to be 0.03 to 0.06 kg/m³ according to approval of a purchaser.

As the method of analyzing the amount of chloride in concrete, after preparing a concrete sample through core collection and crushing, there are various methods, such as a silver nitrate titration method, a silver chromate absorptiometry method, a potentiometric titration method using a chloride ion selective electrode, a chloride ion amount calculation method, a mercury (II) method, an absorbance spectrometry method of mercury (II) thiocyanate, an ion electrode method, a conductivity titration method, and a coulometric titration method.

However, there are not many chloride amount analysis methods that can be actually applied to sites, and in particular, since there are many difficulties in accurately performing the process of taking, processing, and analyzing concrete samples at the site, a simpler method is needed.

Meanwhile, unclassified chemical attack damage scale assessment according to the related art will be described as follows.

Other substances that chemically corrode concrete may be largely divided into two categories. One is the case where a cement hydration product and acids in concrete, animals and plants, inorganic salts, hydrogen sulfide, sulfurous acid gas, and the like cause a chemical reaction to convert the cement hydration product into a soluble substance, which causes concrete to collapse, and the other is the case where sulfate reacts with the cement hydration product in concrete to form a new compound and is accompanied by expansion, and the concrete deteriorates by expansion failure.

In this way, there are many substances that chemically corrode concrete, and in this case, the rate and degree of corrosion are affected by not only the type of corrosive substance, but also presence or absence of concentration, temperature, wet and dry method, presence or absence of abrasion or impact, presence or absence of a flow of corrosive solution, concrete quality, and the like.

The deterioration of concrete by such chemical corrosion takes on various aspects depending on the type or concentration of the deteriorating chemical substance and the difference in environmental conditions. For example, the deterioration by corrosive substances from the outside proceeds from a surface to an inside of concrete. There is a case where the appearance of concrete shows significant signs of deterioration in the early stage of deterioration and a case where the deterioration proceeds to some extent and then signs of deterioration appear on the surface for the first time.

In addition, the signs of concrete deterioration by chemical corrosion on the exterior include exposure of aggregate, surface peeling, detachment of aggregate, pulverization, softening, leaching of rust, leaching of liquid gel, swelling, occurrence of cracks, eruption of crystals, concrete deterioration, exposure of rebar, corrosion, and the like. In addition, the signs of deterioration that can be determined from the appearance include a decrease in mechanical strength, a decrease in modulus of elasticity, expansion, contraction, neutralization, corrosion of rebar, an increase in porosity, and the like.

As other methods of unclassified chemical attack damage scale assessment for a concrete structure, there are X-ray fluorescence analysis, X-ray diffraction analysis, thermal analysis, infrared absorption spectrum analysis, various microscopic analysis methods, and the like. However, in the case of concrete, in addition to cement, various admixtures, aggregates, and water are included, and the amount of sample used for a series of analyses is very small as the level of 0.01 to 1.00 g, so there is a lot of room for error in the final result according to the concrete sampling method, which requires attention.

Meanwhile, as the related art, Korean Patent No. 10-1554165, filed and registered for by the applicant of the present invention, discloses an invention entitled "System for Predicting Residual Service Life of Fire-Damaged Concrete Structures and Method for The Same," the disclosure of which is incorporated herein by reference to form a part of this invention.

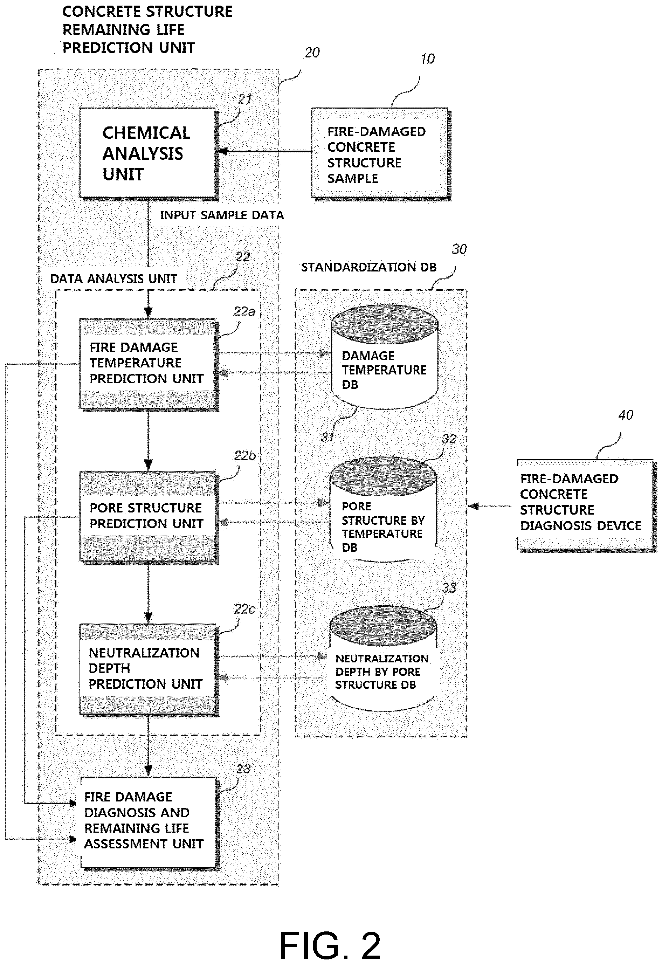

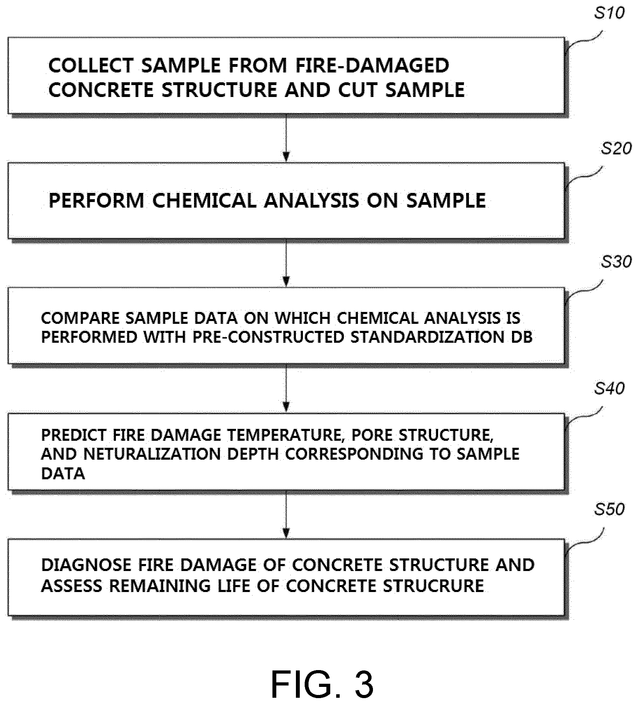

FIG. 2 is a block diagram of a system for predicting a remaining life of a fire-damaged concrete structure according to the related art, and FIG. 3 is an operation flowchart of a method of predicting a remaining life of a fire-damaged concrete structure.

Referring to FIG. 2, the system for predicting a remaining life of a fire-damaged concrete structure according to the related art largely includes a fire-damaged concrete structure sample 10, a concrete structure remaining life prediction unit 20, a standardization database (DB) 30, and a fire-damaged concrete structure diagnosis device 40, in which the concrete structure remaining life prediction unit 20 includes a chemical analysis unit 21, a data analysis unit 22, and a fire damage diagnosis and remaining life assessment unit 23.

The fire-damaged concrete structure sample 10 is collected, from a fire-damaged concrete structure, for example, a structure, by performing drilling up to a target point of a concrete structure using, for example, a mobile core drill, and precisely processed. In this case, a size of the sample 10 may be various, and is preferably 10 mm in diameter × 40 mm in length. In addition, the collected fire-damaged concrete structure sample 10 is precisely cut using various cutters, or the like. For example, the collected fire-damaged concrete structure sample 10 can have various cutting sizes, but may be cut at intervals of 10 mm to prepare a total of 4 samples for each depth.

The concrete structure remaining life prediction unit 20 may acquire sample data by performing chemical analysis on the fire-damaged concrete structure sample 10 processed as a sample for chemical analysis, and compare the sample data with data previously stored in the standardization DB 30 to predict fire damage temperature, a pore structure, and a neutralization depth, respectively, thereby diagnosing fire damage to the fire-damaged concrete structure sample 10 and assessing the remaining life.

Specifically, the chemical analysis unit 21 of the concrete structure remaining life prediction unit 20 acquires the sample data by performing the chemical analysis on the fire-damaged concrete structure sample 10 processed as a sample for chemical analysis.

The data analysis unit 22 of the concrete structure remaining life prediction unit 20 compares the sample data acquired by the chemical analysis unit 21 with the data previously stored in the standardization DB 30 to predict the fire damage temperature, the pore structure, and the neutralization depth, respectively. Here, the data analysis unit 22 may include a fire damage temperature prediction unit 22a, a pore structure prediction unit 22b, and a neutralization depth prediction unit 22c.

Specifically, the fire damage temperature prediction unit 22a of the data analysis unit 22 compares the sample data acquired by the chemical analysis unit 21 with the data stored in the damage temperature DB 31 of the standardization DB 30 to predict the fire damage temperature. In addition, the pore structure prediction unit 22b of the data analysis unit 22 compares the data previously stored in the pore structure by temperature DB 32 of the standardization DB 30 to predict the post structure by temperature. In addition, the neutralization depth prediction unit 22c of the data analysis unit 22 predicts the neutralization depth by comparing with the data previously stored in the neutralization depth by pore structure DB 33 of the standardization DB 30.

The fire damage diagnosis and remaining life assessment unit 23 of the concrete structure remaining life prediction unit 20 diagnoses the fire damage of the fire-damaged concrete structure sample 10 according to the fire damage temperature, the pore structure, and the neutralization depth analyzed by the data analysis unit 22, and assesses the remaining life.

The standardization DB 30 standardizes and stores experimental data obtained through precise experiments using the fire-damaged concrete structure diagnosis device 40, and is used as comparison data for sample data acquired by the chemical analysis unit 21.

Specifically, the standardization DB 30 includes a damage temperature DB 31, a pore structure by temperature DB 32, and a neutralization depth by pore structure DB 33.

The damage temperature DB 31 of the standardization DB 30 stores real-time chemical property change data according to the fire damage temperature through various chemical analyses such as XRD analysis of the fire-damaged concrete structure. In addition, the pore structure by temperature DB 32 stores the pore structure characteristic change data according to the fire damage temperature by pore amount analysis device such as BET analysis and porosimeter analysis of the fire-damaged concrete structure. In addition, the neutralization depth by pore structure DB 33 stores the neutralization depth data according to the pore structure change by the accelerated neutralization analysis of the fire-damaged concrete structure.

The fire-damaged concrete structure diagnosis device 40 may measure a change in characteristics of a sample for each exposure temperature and exposure time and storing each piece of data whenever a heating temperature of a concrete structure test body rises for various chemical analyzes such as XRD, various pore analyses such as brunauer-emmett-teller (BET), and an accelerated neutralization (carbonation) analysis test, and store each piece of data, which may be constructed into the standardization DB 30.

In addition, referring to FIG. 3, in the method of predicting a remaining life of a fire-damaged concrete structure according to the related art, first, the sample 10 is collected from the fire-damaged concrete structure, and the sample 10 is precisely cut and processed (S10), and then chemical analysis (XRD) is performed on the precisely cut sample 10 (S20). Thereafter, the chemically analyzed data is used as first input data.

Next, the sample data subjected to the chemical analysis is compared with the pre-constructed standardized DB 30 (S30). In this case, the standardization DB 30 standardizes and stores experimental data obtained through precise experiments using the fire-damaged concrete structure diagnosis device 40.

Next, the fire damage temperature, the pore structure, and the neutralization depth corresponding to the sample data are predicted (S40), and then, the fire damage of the concrete structure is diagnosed and the remaining life is assessed (S50).

According to the related art, the chemical analysis of the fire-damaged concrete structure sample is performed to acquire the sample data, and the remaining life of the fire-damaged concrete structure may be quickly predicted by comparing the acquired sample data with data previously stored in the standardization DB. In addition, it is possible to accurately and scientifically assess the fire damage level to the fire-damaged concrete structure so that the correct repair and reinforcement of the fire-damaged concrete structure may be performed.

However, in the case of the system for predicting a remaining life of a fire-damaged concrete structure according to the related art, the fire damage temperature prediction unit 22a of the data analysis unit 22 compares the sample data acquired by the chemical analysis unit 21 with the data previously stored in the damage temperature DB 31 of the standardization DB 30 to predict the fire damage temperature. However, it is difficult to accurately predict the fire damage temperature because only a change in chemical composition for each exposure temperature of concrete exposed to high temperature is considered through the experiment, and thus, there is a problem in that a predictive rate of fire damage temperature is lowered, and a limitation that it may be applied only to the fire-damaged concrete structure.

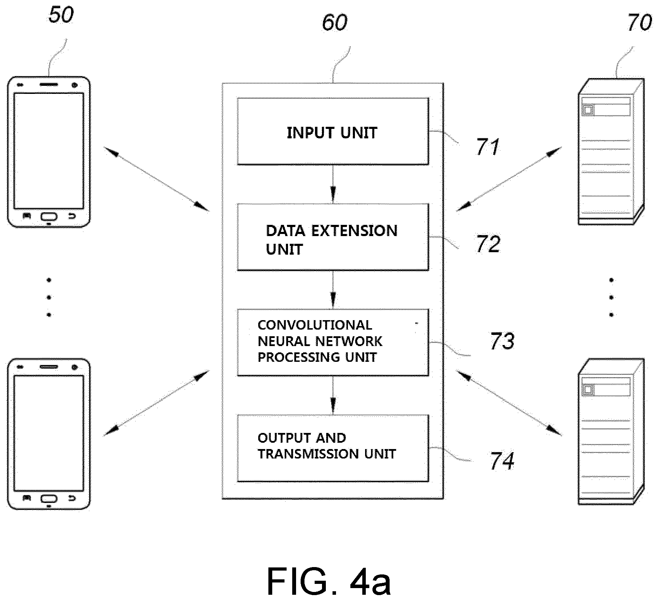

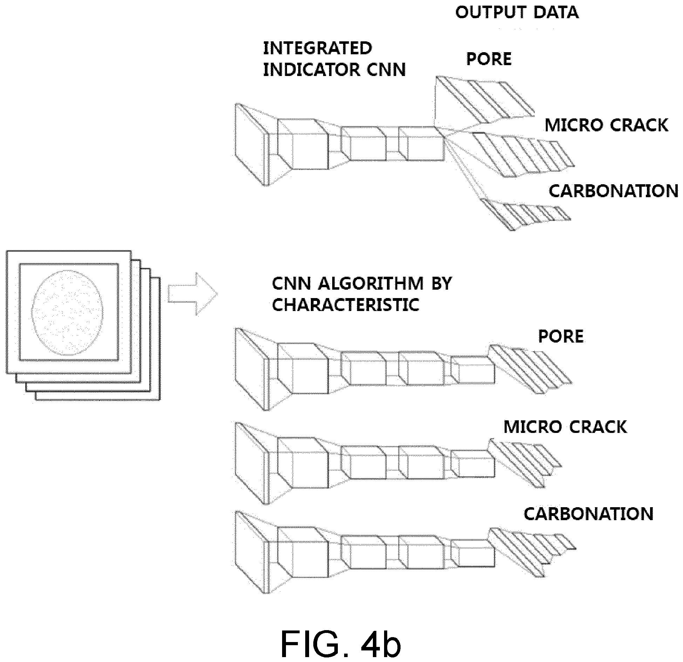

Meanwhile, as another related art, Korean Patent Laid-Open Publication No. 2019-0061515 discloses an invention entitled "System and Method for Evaluating Concrete Structure Degradation Using Deep Neural Networks," which will be described with reference to FIGS. 4A and 4B.

FIG. 4A is a block diagram of a system for time-dependent assessment of concrete deterioration using a deep neural network according to the related art, and FIG. 4B is a classification example according to the number of convolutional neural network models.

Referring to FIG. 4A, the system for time-dependent assessment of concrete deterioration 1 a deep neural network according to the related art includes an input terminal 50 that captures and transmits an image or video of an assessment target structure at the site, and an assessment server 60 that extracts and expands feature data from some images or images of concrete structures captured through the input terminal 50, performs deterioration assessment by deep learning method through a deep neural network, and generates a report and transmits the generated report to the related institution server 70.

In this case, the assessment server 60 includes an image input unit 61 that receives and inputs an image or video transmitted from the input terminal 50, a data extension unit 62 that generates a new image by processing the image or video input through the image input unit 61 into an image suitable for assessment, a convolutional neural network processing unit 63 that receives the new image of the data extension unit 62 and assesses a facility deterioration class, and an output and transmission unit 64 that writes and transmits an assessment result of the convolutional neural network processing unit 63 as a report.

The system for time-dependent assessment of concrete deterioration using a deep neural network according to the related art may use a single convolutional neural network model trained by integrating a pore, strength, a chloride diffusion coefficient, soundness, and the like used for concrete structure performance assessment to perform the performance assessment of the structure using the input image. For example, as illustrated in FIG. 4B, multiple convolutional neural network models may be used.

In other words, by using the model trained by integrating the pore, strength, chloride diffusion coefficient, soundness, and the like used for the structure performance assessment, and a separate convolutional neural network model by deterioration indicator such as bending, microcracks, and carbonization, it is possible to pre-classify the input image for each deterioration indicator and then perform performance assessment. In this way, when multiple models are used, each model may store images capable of determining the degree of deterioration for each indicator and determine the degree of deterioration by indicator by comparing the pre-classified images.

According to the system for time-dependent assessment of concrete deterioration using a deep neural network according to the related art, an image or video of a concrete structure captured at the site where facilities are located is received, and determined by comparing with standard images using a deep neural network to perform the performance assessment of the corresponding facility, thereby performing more objective and reliable assessment of concrete deterioration. In addition, it is possible to assess a degree of deterioration for each characteristic, such as pores, microcracks, and carbonation, only by inputting the image or video, and it is possible to automatically generate and output reports on assessment results, thereby establishing a systematic and efficient assessment work system.

However, the system for time-dependent assessment of concrete deterioration using a deep neural network according to the related art is a scheme of extracting and expanding feature data from some images or videos of a concrete structure captured through an input terminal, and performing deterioration assessment with a deep learning method through a deep neural network, and has a problem in that accuracy is lowered because actual concrete structure samples are not used.

As described above, it is very important to integrally assess a fire damage scale, strength development, a carbonation damage scale, a salt damage scale, and a damage scale caused by other corrosive gases, acids, salts, and the like for a concrete structure to confirm the quality of concrete and level of concrete deterioration.

In other words, concrete is a compound, and changes in chemical compositions that constitute concrete affect the quality change and deterioration level of concrete. The most scientific and quantitative assessment method of concrete is a method of investigating chemical compositions of concrete, but it is known that concrete deterioration assessment methods according to the related art are difficult to apply directly to the sites.

### [Disclosure]

### [Technical Problem]

The present invention provides a deep learning-based system for time-dependent assessment of concrete deterioration and a method for the same capable of assessing, based on a deep learning-based chemical analysis, deteriorations of a concrete structure, such as fire damage scale assessment, strength development assessment, carbonation damage scale assessment, salt damage scale assessment, and unclassified chemical attack damage scale assessment of the concrete structure, using a forensic investigation of concrete structure (FIS) analysis tool capable of performing a practical sampling design (PSD) process, an experimental design (ED) process, a process of applying a chemical profiling algorithm (CPA), and a process of applying a deep learning interface algorithm (DIA).

In addition, the present invention provides a deep learning-based system for time-dependent assessment of concrete deterioration and a method for the same capable of expanding to and assessing not only sample collection but also a member unit or an entire structure unit by synthesizing analysis results of concrete structure samples collected from a specific location at the site using statistical data in other field investigation data, standardized experimental data, history information, and the like in a big data storage unit, in a process of applying a DIA of an FIS analysis tool for assessing a deterioration damage scale of a concrete structure, and optimizing the assessed results.

In addition, the present invention provides a deep learning-based system for time-dependent assessment of concrete deterioration and a method for the same capable of correctly diagnosing a deterioration of a concrete structure by performing physical and chemical calibrations for contamination sources on a concrete structure sample to increase reliability of deterioration assessment results of the concrete structure, clearly assessing the deterioration damage scale of the concrete structure according to a depth change, and predicting future behavior as a function of time.

### [Technical Solution]

According to an exemplary embodiment, a deep learning-based system for time-dependent assessment of concrete deterioration includes: a practical sampling design (PSD) unit that performs practical sampling on a concrete structure to determine a sampling location and quantity from an on-site concrete structure, collect a sample, process the collected sample into an optimal sample for analysis, and store the optimal sample for analysis; a sample processing unit that physically separates and processes a primary constituent materials of the concrete structure sample; an experimental design (ED) unit that performs chemical analysis on the concrete structure sample processed in the sample processing unit using at least one chemical analysis device; a chemical profiling algorithm (CPA) application unit that chemically separates a secondary constituent material of the concrete structure sample and applies CPA for coding a chemical analysis result; a deep learning interface algorithm (DIA) application unit that applies a DIA to an analysis result of the ED unit to compare with pre-constructed standardized experimental data and derive an optimal analysis result; and a deterioration assessment unit that assesses a deterioration damage scale of the concrete structure sample, and includes fire damage scale assessment, strength development assessment, carbonation damage scale assessment, salt damage scale assessment, and unclassified chemical attack damage scale assessment for the concrete structure sample.

The deep learning-based system for time-dependent assessment of concrete deterioration may further include a forensic investigation of concrete structure (FIS) analysis tool configured to scientifically quantitatively assess a deterioration damage scale from the collection of the concrete structure sample in association with the PSD unit, the ED unit, the CPA application unit, and the DIA application unit.

The deep learning-based system for time-dependent assessment of concrete deterioration may further include a time dependent assessment (TDA) derivation unit that implements simulation to predict a deterioration progress rate of the concrete structure according to the deterioration assessment result of the deterioration assessment unit as a function of time t to derive key information, assess deterioration damage scale by cause, and code the deterioration damage scale.

The ED unit may perform the chemical analysis using at least one of X-ray diffraction (XRD), X-ray fluorescence (XRF), Fourier transform infrared (FTIR), nuclear magnetic resonance (NMR), or thermogravimetric analysis (TGA).

The CPA application unit may include: a TGA unit (141) that performs TGA measuring a mass reduction rate of the concrete structure sample; a FTIR analysis unit that analyzes a maximum value of a peak appearing at a specific wavelength number and an area of a corresponding part according to a fire damage temperature of the concrete structure sample; and a chemical analysis unit that performs chemical analysis using various chemical analysis devices in parallel according to a purpose of analysis.

The higher the fire damage temperature in the TGA unit, the smaller the mass loss pattern and the total mass reduction rate may appear, and the higher the overall fire damage temperature in the Fourier transform infrared analysis unit, the smaller the maximum value of the peak and the area of the part.

The DIA application unit may include: a first DIA application unit that uses a single analysis result of the TGA unit as input data to derive a final output for calculating a fire damage temperature for each damage depth inside a fire-damaged concrete structure and a fire temperature curve of a part corresponding to a heat source outside the concrete structure; a second DIA application unit that, when a correlation value derived from the first DIA application unit is below an appropriate level, uses an analysis result of the TGA unit, an analysis result of the FTIR analysis unit, and an analysis result of the chemical analysis unit as input data to weight and re-analyze a clearly appearing feature, and derives a final output for calculating the fire damage temperature for each damage depth inside the concrete structure and the fire temperature curve of the part corresponding to the heat source outside the concrete structure; and a data comparison and analysis unit that compares an analysis result of the first DIA application unit or an analysis result of the second DIA application unit with the pre-constructed standardized experimental data, in which the feature of the second DIA application unit may be a clearly appearing result value at a specific temperature.

The DIA application unit may compare and matches the analyzed data of the concrete structure sample collected from a site with pre-constructed statistical data, and apply a wholistic approach process that expands a target to be analyzed to the entire concrete structure as well as a sample location of the concrete structure sampled at the site and an optimization process to obtain a result at a location where sampling was not performed during the wholistic approach process.

The deep learning-based system for time-dependent assessment of concrete deterioration may further include a big data storage unit that stores other field investigation data, standardized experimental data, and history information, in which the standardized experimental data refers to a storage space that includes a TGA result indicating a mass reduction rate for each exposure temperature of the concrete sample under standardized laboratory conditions, an FTIR analysis result indicating a change in chemical composition for each exposure temperature, and other various chemical analysis results, the other field investigation data refers to a storage space that includes information obtained through various interviews, closed-circuit television (CCTV) analysis, and communication obtained through a corresponding fire site investigation, the history information refers to a storage space that includes information organized by converting a fire site analysis result generated in the meantime into data, and the big data storage unit comprehensively includes all such information.

The deep learning-based system for time-dependent assessment of concrete deterioration may further include a sample contamination source calibration unit that removes a contaminant contaminating the concrete structure sample or calibrates a contamination cause in order to increase accuracy of an analysis result of the concrete structure sample.

The sample contamination source calibration unit may include: a sample contamination source physical calibration unit that calibrates the concrete structure sample by physically removing a contamination source of the concrete structure sample when the sample processing unit processes the concrete structure sample; and a sample contamination source chemical calibration unit that calibrates the concrete structure sample by chemically removing the contamination source of the concrete structure sample when the CPA of the CPA application unit is applied.

The sample contamination source physical calibration unit may calibrate the concrete structure sample by physically removing the contaminant contaminating the concrete structure sample using a centrifuge, and physically separate each constituent material of the concrete structure sample collected from a site using the centrifuge and then perform this experiment only on the targeted concrete structure sample to derive a final result.

The sample contamination source chemical calibration unit may cross-confirm whether the contamination source is included using one or more chemical analysis devices, and then, when the contamination source is included, removes the contamination source to derive a calibrated value as the final value or select only results of some sections or specific chemical compositions from a chemical analysis result using single device to be derived as the final result.

The deterioration assessment unit may include: a fire damage scale assessment unit that predicts fire damage temperature for each depth of the concrete structure and predicts a fire damage temperature curve of a surface of the concrete structure; a strength development assessment unit that assesses a strength development level according to hydration delay and chemical damage for each depth of the concrete structure; a carbonation damage scale assessment unit that investigates a carbonation damage scale for each depth of the concrete structure and predicts a carbonation damage rate; a salt damage scale assessment unit (174) that investigates a chloride damage scale for each depth of the concrete structure and predicts a salt damage rate; and an unclassified chemical attack damage scale assessment unit that investigates a damage scale caused by corrosive gas and damage caused by salts of sulfate and acid for each depth of the concrete structure and predicts unclassified chemical damage deterioration rates.

The fire damage scale assessment unit may include: a fire damage temperature prediction unit that predicts fire damage temperature at a specific location for each damage depth of a fire-damaged concrete structure according to the DIA application unit; a fire temperature curve calculation unit that calculates a fire temperature curve corresponding to a first heat source outside the fire-damaged concrete structure according to the DIA application unit; a thermal diffusion path prediction unit that predicts a thermal diffusion path inside the fire-damaged concrete structure according to the predicted fire damage temperature and the calculated fire temperature curve; and a flame movement path tracking reproduction unit that tracks and reproduces a heat movement path from a fire location of a fire site as a function of time according to the predicted fire damage temperature and the calculated fire temperature curve, and the thermal diffusion path prediction unit identifies the thermal diffusion path over time in response to the prediction of the fire damage temperature at the time of the fire for an unknown fire-damaged concrete structure sample collected from the fire site.

The fire temperature curve calculation unit may collect samples for each depth from the same location, compare and analyze each piece of data analyzed in units of one set, and expresses the data as a function of time t to calculate the fire temperature curve of the surface corresponding to the first heat source of the concrete structure, and the fire temperature curve is given by a magnitude of a hydrothermal temperature over time and a value of the exposure time.

The concrete structure sample may be collected by drilling or coring a building member of a wall, a column, or a slab of a fire-damaged concrete structure, in which the drilling or coring may be performed up to a location of a main rebar with a minimum diameter and a depth of at least 40 mm.

According to another exemplary embodiment, a deep learning-based method of time-dependent assessment of concrete deterioration includes: a) as a PSD process, performing practical sampling on a concrete structure to collect a concrete structure sample from a site, process the concrete structure sample into a sample for analysis in an optimal state, and store the processed concrete structure sample; b) physically separating and processing primary constituent materials of the concrete structure sample; c) as an ED process, performing chemical analysis on the processed concrete structure sample using at least one chemical analysis device; d) as a process of applying a CPA, chemically separating a secondary constituent material of the concrete structure sample and coding a chemical analysis result; e) as a process of applying a DIA, after applying the DIA to the chemical analysis result for deep learning analysis, deriving an optimal analysis result by comparing with pre-constructed standardized experimental data; and f) assessing the deterioration of the concrete structure sample, in which in operation f), fire damage, strength development, a carbonation damage scale, a salt damage scale, and an unclassified chemical attack damage scale are assessed for the concrete structure sample.

The deep learning-based method of time-dependent assessment of concrete deterioration may further include g) implementing simulation to predict the deterioration progress rate of the concrete structure as a function of time t according to the deterioration assessment result, deriving key information, assessing and coding a deterioration damage scale by each cause.

The deep learning-based method of time-dependent assessment of concrete deterioration may further include h) storing a deterioration assessment result of the concrete structure sample in a big data storage unit.

The deep learning-based method of time-dependent assessment of concrete deterioration may further include i) in order to increase the accuracy of the analysis result of the concrete structure sample, removing contaminants contaminating the concrete structure sample or calibrating the cause of contamination.

### [Advantageous Effects]

According to the present invention, it is possible to assess, most concrete structure deterioration through deep learning-based chemical analysis such as fire damage scale assessment, strength development assessment, carbonation damage scale assessment, salt damage scale assessment, and other chemical attack damage scale assessment of the concrete structure, using a forensic investigation of concrete structure (FIS) analysis tool capable of performing a practical sampling design (PSD) process, an experimental design (ED) process, a process of applying a chemical profiling algorithm (CPA), and a process of applying a deep learning interface algorithm (DIA).

According to the present invention, it is possible to increase a predictive rate by expanding and assessing not only a sample collection location but also expanding to and assessing a member unit or an entire structure unit by synthesizing statistical data in other field investigation data, standardized experimental data, history information, and the like in a big data storage unit with an analysis result of a concrete structure sample collected from a specific location at the site, in a process of applying a DIA of an FIS analysis tool for a concrete structure, and optimizing the assessed results.

According to the present invention, it is possible to correctly diagnose the deterioration of a concrete structure by performing physical and chemical calibration for contamination sources in a concrete structure sample to increase the reliability of deterioration assessment results of the concrete structure, clearly assessing the deterioration damage scale of the concrete structure according to a depth change, and predicting future behavior as a function of time, thereby achieving reasonable repair and reinforcement of the concrete structure.

According to the present invention, it is possible to predict the deterioration progress rate of a concrete structure as a function of time, and in particular, it is possible to implement a simulation by expanding to member units and entire structure units, secure the highest predictive rate compared to the existing method, and achieve the fastest analysis.

According to the present invention, it is possible to simultaneously assess most deterioration levels related to a concrete structure, and monitor the post-quality of the concrete structure from immediately after completion to demolition.

### [Description of Drawings]

FIG. 1 is a diagram illustrating that the compressive strength of a concrete structure decreases as the temperature rises during a fire.
FIG. 2 is a configuration diagram of a system for predicting the remaining life of a fire-damaged concrete structure according to the related art.
FIG. 3 is an operation flowchart of a method of predicting the remaining life of a fire-damaged concrete structure according to the related art.
FIG. 4A is a block diagram of a system for time-dependent assessment of concrete deterioration using a deep neural network according to the related art, and FIG. 4B is a classification example according to the number of convolutional neural network models.
FIG. 5 is a configuration diagram of a deep learning-based system for time-dependent assessment of concrete deterioration according to an embodiment of the present invention.
FIG. 6 is a diagram illustrating the concept of a forensic investigation of concrete structure (FIS) analysis tool, which is the deep learning-based system for time-dependent assessment of concrete deterioration according to the embodiment of the present invention.
FIG. 7 is a detailed configuration diagram of a deterioration assessment unit in the deep learning-based system for time-dependent assessment of concrete deterioration according to the embodiment of the present invention.
FIG. 8 is a configuration diagram specifically illustrating a deterioration assessment unit that performs fire damage scale assessment in the deep learning-based system for time-dependent assessment of concrete deterioration according to the embodiment of the present invention.
FIG. 9 is a configuration diagram of a big data storage unit in the deep learning-based system for time-dependent assessment of concrete deterioration according to the embodiment of the present invention.
FIG. 10 is a diagram illustrating an analysis result of a thermogravimetric analysis (TGA) unit, which is standardized experimental data of the big data storage unit in the deep learning-based system for time-dependent assessment of concrete deterioration according to the embodiment of the present invention.
FIG. 11 is a diagram illustrating a Fourier transform infrared (FTIR) analysis result for constructing the standardized experimental data of the big data storage unit in the deep learning-based system for time-dependent assessment of concrete deterioration according to the embodiment of the present invention.
FIG. 12 is a diagram illustrating a nuclear magnetic resonance (NMR) measurement result in the deep learning-based system for time-dependent assessment of concrete deterioration according to the embodiment of the present invention.
FIG. 13 is a diagram illustrating a surface of a concrete structure after sampling as a practical sampling design (PSD) process in the deep learning-based system for time-dependent assessment of concrete deterioration according to the embodiment of the present invention.
FIG. 14 is a photograph showing the physical removal of contaminants by centrifugation as a sample contamination source physical calibration in the deep learning-based system for time-dependent assessment of concrete deterioration according to the embodiment of the present invention.
FIG. 15 is a diagram illustrating the removal of a contaminated section after TGA as sample contamination source chemical calibration in the deep learning-based system for time-dependent assessment of concrete deterioration according to the embodiment of the present invention.
FIG. 16 is a diagram illustrating the selection and analysis of a specific section in which calcium silica hydrate (C-S-H) is present after NMR Si analysis as sample contamination source chemical calibration in the deep learning-based system for time-dependent assessment of concrete deterioration according to the embodiment of the present invention.
FIG. 17 is a diagram schematically illustrating a deep learning interface algorithm (DIA) applied to the deep learning-based system for time-dependent assessment of concrete deterioration according to the embodiment of the present invention.
FIG. 18 is a diagram illustrating the selection of a DIA with high predictive power by searching for a DIA applicable to the deep learning-based system for time-dependent assessment of concrete deterioration according to the embodiment of the present invention.
FIG. 19 is a diagram illustrating a first DIA application unit in the deep learning-based system for time-dependent assessment of concrete deterioration according to the embodiment of the present invention.
FIG. 20 is a diagram illustrating a second DIA application unit in the deep learning-based system for time-dependent assessment of concrete deterioration according to the embodiment of the present invention.
FIG. 21 is an operation flowchart of a deep learning-based method of time-dependent assessment of concrete deterioration according to an embodiment of the present invention.

### [Best mode of the Invention]

DEEP LEARNING-BASED SYSTEM FOR TIME-DEPENDENT ASSESSMENT OF CONCRETE DETERIORATION, AND METHOD FOR THE SAME according to the present invention include: A deep learning-based system for time-dependent assessment of concrete deterioration, comprising: a practical sampling design (PSD) unit (110) that performs practical sampling on a concrete structure to determine a sampling location and quantity from an on-site concrete structure, collect a sample, process the collected sample into an optimal sample for analysis, and store the optimal sample for analysis; a sample processing unit (120) that physically separates and processes primary constituent materials of the concrete structure sample; an experimental design (ED) unit (130) that performs chemical analysis on the concrete structure sample processed in the sample processing unit (120) using at least one chemical analysis device; a chemical profiling algorithm (CPA) application unit (140) that chemically separates a secondary constituent material of the sample of the concrete structure and applies CPA for coding a chemical analysis result; a deep learning interface algorithm (DIA) application unit (150) that applies a DIA to an analysis result of the ED unit (130) to compare with pre-constructed standardized experimental data and derive an optimal analysis result; and a deterioration assessment unit (170) that assesses a deterioration damage scale of the sample of the concrete structure, and includes fire damage scale assessment, strength development assessment, carbonation damage scale assessment, salt damage scale assessment, and unclassified chemical attack damage scale assessment for the sample of the concrete structure.

### [Modes of the Invention]

Hereinafter, embodiments of the present invention will be described in detail with reference to the accompanying drawings so that those skilled in the art to which the present invention pertains may easily practice the present invention. However, the present invention may be implemented in various different forms, and is not limited to embodiments described herein. In addition, in the drawings, portions unrelated to the description will be omitted to clearly describe the present disclosure, and similar portions will be denoted by similar reference numerals throughout the specification.

Throughout the present specification, unless explicitly described to the contrary, "comprising" any components will be understood to imply the inclusion of other elements rather than the exclusion of any other elements. In addition, the terms "...unit," and the like described in the specification means a processing unit of at least one function or operation and may be implemented by hardware or software or a combination of hardware and software.

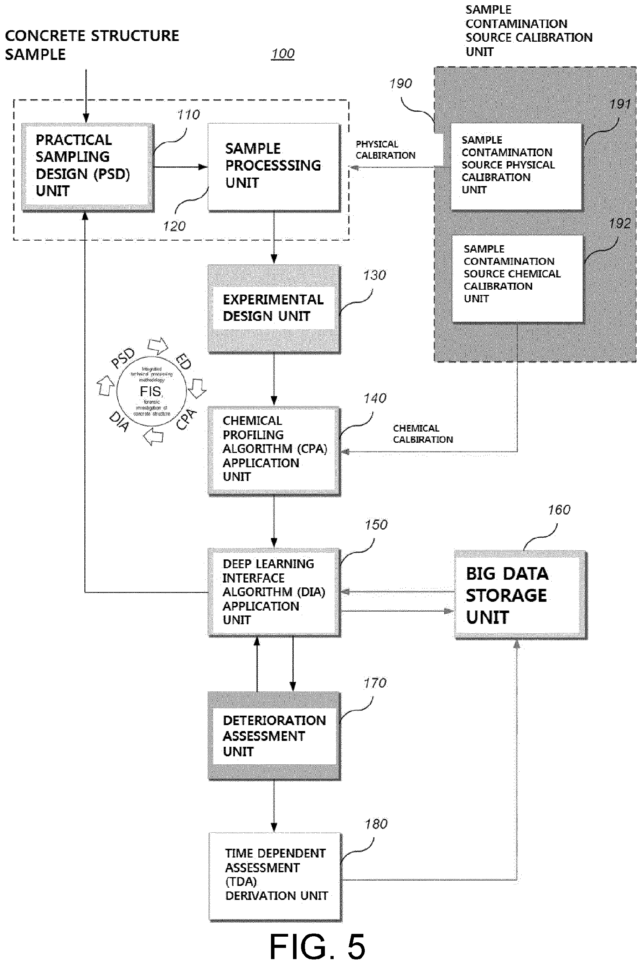

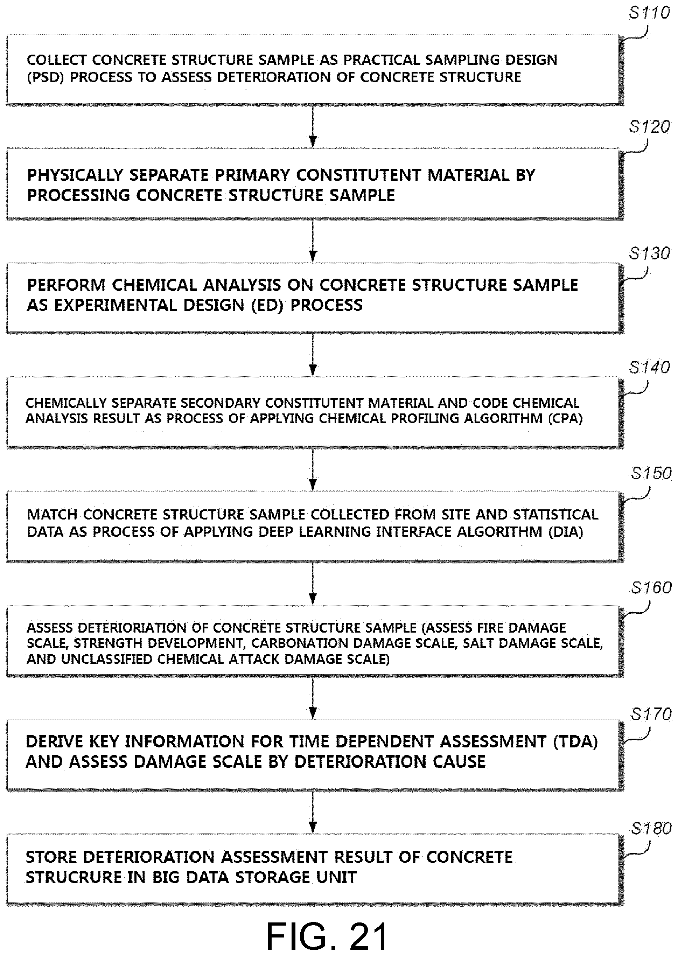

Hereinafter, a deep learning-based system for time-dependent assessment of concrete deterioration according to an embodiment of the present invention will be described with reference to FIGS. 5 to 20. In addition, a deep learning-based method of time-dependent assessment of concrete deterioration according to an embodiment of the present invention will be described with reference to FIG. 21.

### [Deep Learning-Based System 100 for Time-Dependent Assessment of Concrete Deterioration]

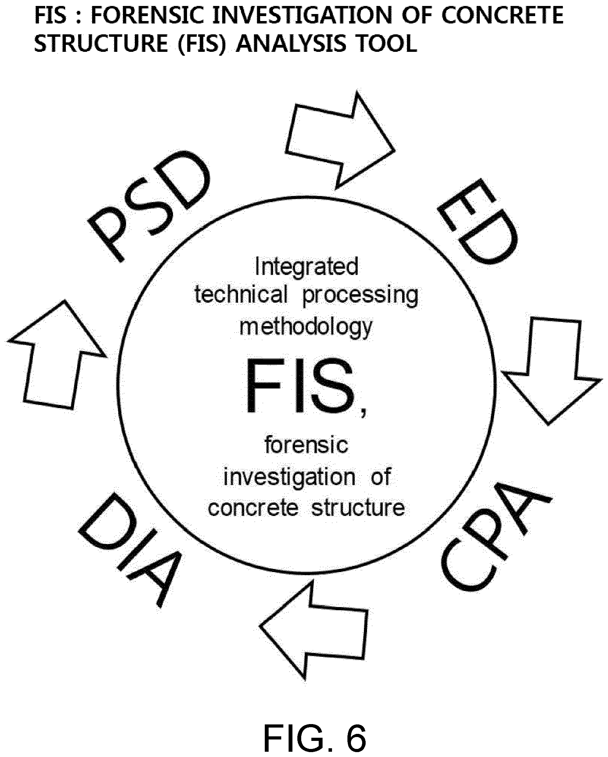

FIG. 5 is a configuration diagram of a deep learning-based system for time-dependent assessment of concrete deterioration according to an embodiment of the present invention, and FIG. 6 is a diagram illustrating a concept of a forensic investigation of concrete structure (FIS) analysis tool, which is the deep learning-based system for time-dependent assessment of concrete deterioration according to the embodiment of the present invention.

Referring to FIGS. 5 and 6, the deep learning-based system 100 for time-dependent assessment of concrete deterioration according to the embodiment of the present invention is an FIS analysis tool, and includes a practical sampling design (PSD) unit 110, a sample processing unit 120, an experimental design (ED) unit 130, a chemical profiling algorithm (CPA) application unit 140, a deep learning interface algorithm (DIA) application unit 150, a big data storage unit 160, a deterioration assessment unit 170, a time dependent assessment (TDA) derivation unit 180, and a sample contamination source calibration unit 190. In this case, the sample contamination source calibration unit 190 may include a sample contamination source physical calibration unit 191 and a sample contamination source chemical calibration unit 192.

The PSD unit 110 performs practical sampling on a concrete structure so that a concrete structure sample is collected from the site, processed into a sample for analysis in an optimal state, and stored. In this case, the concrete structure sample is collected by drilling or coring the building members of a wall, a column, or a slab of a fire-damaged concrete structure, in which the drilling or coring is performed up to a location of the main rebar with a minimum diameter and a depth of at least 40 mm. In this case, it is preferable to sample the fire-damaged concrete structure in a minimum amount so as not to damage the fire-damaged concrete structure.

In this case, when trying to minimize the diameter of the drilling or coring, the fire-damaged concrete structure sample may be collected through one or more samplings in close proximity to secure the representativeness of the sampling location and the required amount.

In addition, the fire-damaged concrete structure sample defines, as one set, two or more samples according to the depth change at one location, and all analyses are performed in units of one set, and an output may be derived by finding all the corresponding values or the most similar values. Accordingly, when comparing result values for each section, a thermal diffusion rate may be expressed as a function of time.

As will be described later, after investigating the correlation with the high temperature exposure temperature through various chemical and physical experiments such as chemical analysis and a mass reduction rate and organizing how the chemical composition and physical characteristics of the inside of the fire-damaged concrete structure sample change at a specific temperature, the fire-damaged concrete structure sample collected from the site may be easily found by comparing specific values during the process of finding data, and in this case, the prestored data is defined as standardized experimental data.

The sample processing unit 120 physically separates and processes primary constituent materials of the concrete structure sample.

The ED unit 130 performs chemical analysis on the concrete structure sample processed by the sample processing unit 120 using at least one chemical analysis device. In this case, the ED unit 130 may perform the chemical analysis on the concrete structure sample collected and processed at the site using at least one of X-ray diffraction (XRD), X-ray fluorescence (XRF), Fourier transform infrared (FTIR), nuclear magnetic resonance (NMR), or thermogravimetric analysis (TGA).

The CPA application unit 140 chemically separates secondary constituent materials of the concrete structure sample and applies the CPA that codes the chemical analysis results.

The DIA application unit 150 applies the DIA to the analysis result of the ED unit 130 for deep learning analysis, and then compares the analysis result with pre-constructed standardized experimental data to derive optimal analysis results.

The big data storage unit 160 stores other field investigation data, standardized experimental data and history information, and expands to assess a member unit or an entire structure unit by synthesizing the analysis results of the concrete structure samples collected from specific locations at the site with statistical data such as other field investigation data, standardized experimental data, history information, and the like in the big data storage unit 160, in the process of applying the DIA.

The deterioration assessment unit 170 assesses the deterioration of the concrete structure sample. For example, the deterioration assessment unit 170 may perform fire damage scale assessment, strength development assessment, carbonation damage scale assessment, salt damage scale assessment and unclassified chemical attack damage assessment on the concrete structure sample.

The TDA derivation unit derivation unit 180 may derive key information by implementing simulation to predict a deterioration progress rate of the concrete structure as a function of time t according to the deterioration assessment result of the deterioration assessment unit 170 and assess and code a deterioration damage scale by cause, and the result of the coding process of the TDA derivation unit 180 may be stored in the big data storage unit 160 and constructed into a database (DB).

The sample contamination source calibration unit 190 includes a sample contamination source physical calibration unit 191 and a sample contamination source chemical calibration unit 192, and in order to increase the accuracy of the analysis result of the concrete structure sample, removes contaminants contaminating the concrete structure sample or physically or chemically calibrates the contamination causes.

In the deep learning-based system 100 for time-dependent assessment of concrete deterioration according to the embodiment of the present invention, as illustrated in FIG. 6, the FIS analysis tool is implemented by the PSD unit 110, the ED unit 130, the CPA application unit 140, and the DIA application unit 150, and most of the deteriorations of the concrete structure may be assessed by analyzing and assessing the concrete structure sample collected from the site by the FIS analysis tool.

Here, forensic investigation means sequentially performing cross analysis on the concrete structure samples collected from the site with several tools, for example, the PSD unit, the ED unit, the CPA application unit, and the DIA application unit.

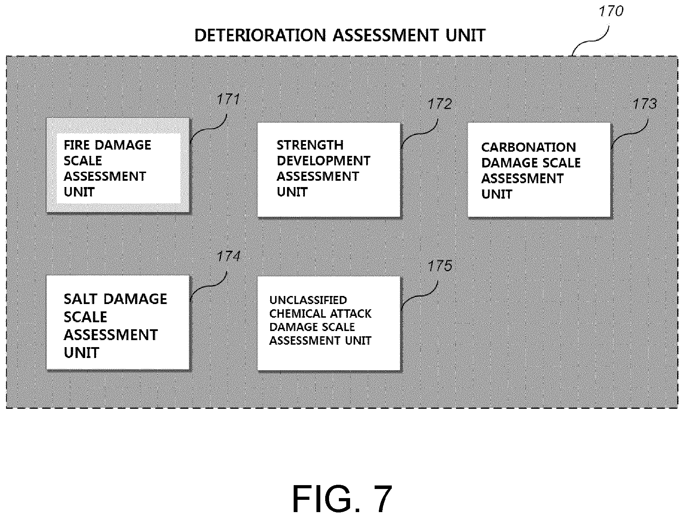

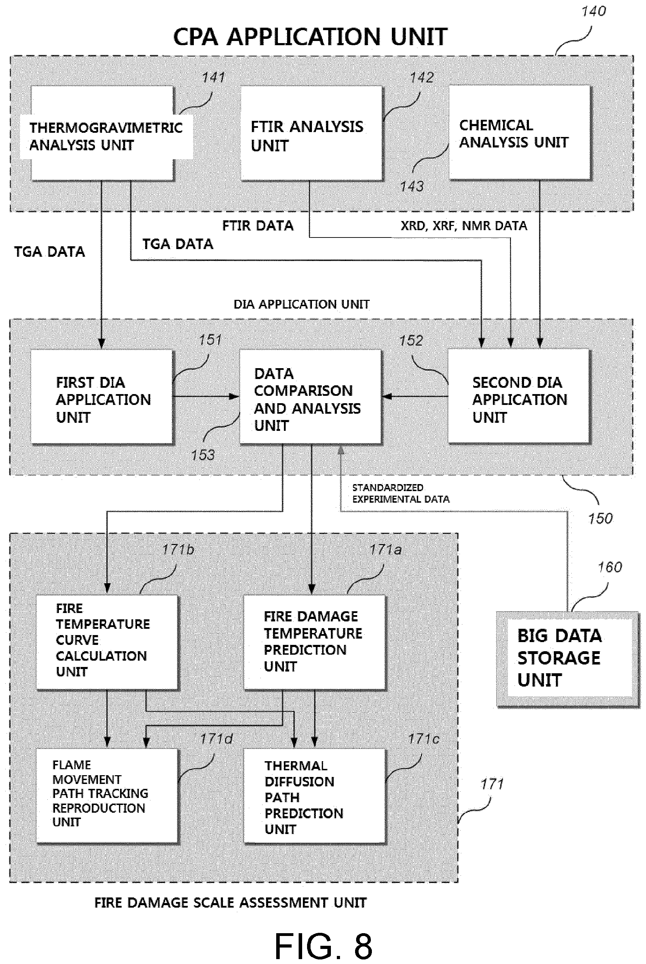

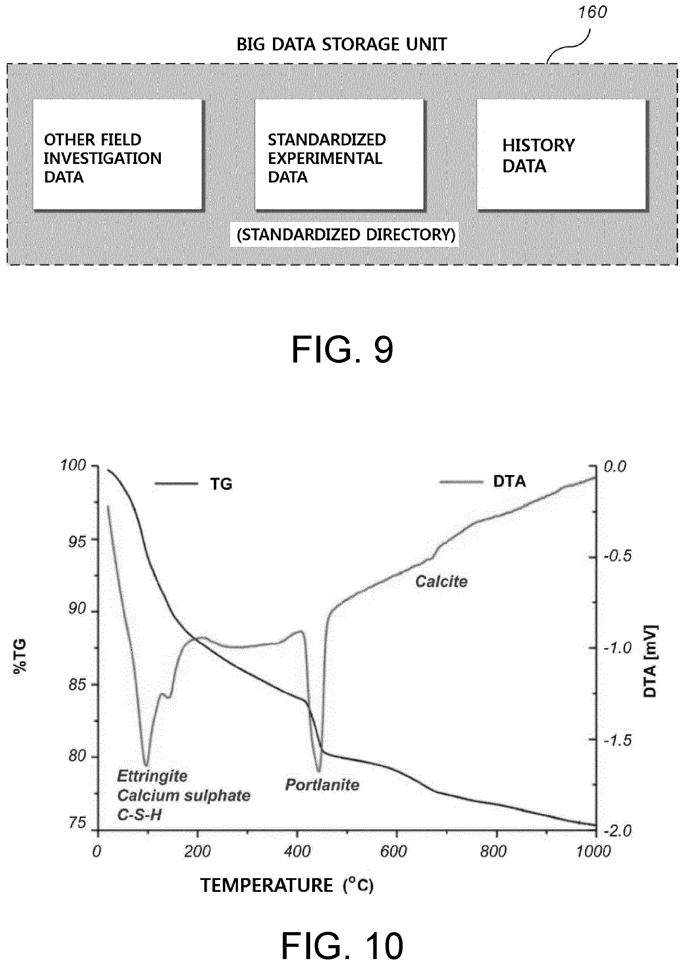

FIG. 7 is a detailed configuration diagram of a deterioration assessment unit in the deep learning-based system for time-dependent assessment of concrete deterioration according to the embodiment of the present invention, FIG. 8 is a configuration diagram illustrating in detail the deterioration assessment unit that performs fire damage scale assessment in the deep learning-based system for time-dependent assessment of concrete deterioration according to the embodiment of the present invention, and FIG. 9 is a configuration diagram of a big data storage unit in the deep learning-based system for time-dependent assessment of concrete deterioration according to the embodiment of the present invention.

Referring to FIG. 7, in the deep learning-based system 100 for time-dependent assessment of concrete deterioration according to the embodiment of the present invention, the deterioration assessment unit 170 includes a fire damage scale assessment unit 171, a strength development assessment unit 172, a carbonation damage scale assessment unit 173, a salt damage scale assessment unit 174, and an unclassified chemical attack damage scale assessment unit 175.

The fire damage scale assessment unit 171 predicts the fire damage temperature for each depth of the concrete structure and predicts a fire damage temperature curve of a surface portion of the concrete structure. Accordingly, the deterioration of the concrete structure may be correctly diagnosed by clearly assessing the deterioration damage scale of the concrete structure according to the depth change.

The strength development assessment unit 172 assesses the strength development level according to the hydration delay and chemical damage for each depth of the concrete structure. Accordingly, whether or not the strength of the already-cast concrete at the site has been developed may be quickly diagnosed. For example, it is possible to check whether hydration products are stably present in the cement paste inside the concrete at the site.

The carbonation damage scale assessment unit 173 investigates the carbonation damage scale for each depth of the concrete structure and predicts the carbonation damage rate. Accordingly, it is possible to quantitatively assess the state of the entire member or the entire structure, check the location of the concrete structure where a concrete structure sample has not been collected, accurately predict the carbonation rate, and accurately predict the remaining life.

The salt damage scale assessment unit 174 investigates chloride damage scale for each depth of the concrete structure and predicts the salt damage rate. Accordingly, the salt damage may be easily assessed in the actual field.

The unclassified chemical attack damage scale assessment unit 175 investigates the damage scale caused by corrosive gas for each depth of the concrete structure, the damage caused by salts of sulfate and acid, and predicts the deterioration rate of unclassified chemical damages. Accordingly, it is possible to easily assess unclassified chemical attack damages, such as other corrosive gases, acids, and salts, occurring in the material aspect of concrete.

Specifically, referring to FIG. 8, in the deterioration assessment unit that performs fire damage scale assessment in the deep learning-based system for time-dependent assessment of concrete deterioration according to the embodiment of the present invention, the fire damage scale assessment unit 171 includes a temperature prediction unit 171a, a fire temperature curve calculation unit 171b, a thermal diffusion path prediction unit 171c, and a flame movement path tracking reproduction unit 171d. In this case, the CPA application unit 140 includes a thermogravimetric analysis (TGA) unit 141, an FTIR analysis unit 142, and a chemical analysis unit 143. In addition, the DIA application unit 150 includes a first DIA application unit 151, a second DIA application unit 152, and a data comparison and analysis unit 153.

Specifically, the TGA unit 141 of the CPA application unit 140 performs the TGA unit analysis that measures the mass reduction pattern and the mass reduction rate of the concrete structure sample.

The FTIR analysis unit 142 of the CPA application unit 140 analyzes a maximum value of a peak appearing at a specific wavelength number and an area of the corresponding part according to the fire damage temperature of the concrete structure sample.

The chemical analysis unit 143 of the CPA application unit 140 performs chemical analysis in parallel with a chemical analysis device according to the purpose of analysis.

In this case, the higher the fire damage temperature in the TGA unit 141, the smaller the total mass reduction rate appears, and the higher the overall fire damage temperature in the FTIR analysis unit 142, the smaller the maximum value of the peak and the smaller the area of the part.

FIG. 10 is a diagram illustrating an analysis result of a TGA unit, which is standardized experimental data of the big data storage unit in the deep learning-based system for time-dependent assessment of concrete deterioration according to the embodiment of the present invention.

In the case of the deep learning-based system 100 for time-dependent assessment of concrete deterioration according to the embodiment of the present invention, as illustrated in FIG. 10, the standardized experimental data is acquired through the TGA unit analysis. For example, the standardized experimental data may be stored as a standardized directory in the big data storage unit 160, but is not limited thereto.

Specifically, as illustrated by a red line in FIG. 10, the existing TGA analysis may confirm that mass reduction occurs rapidly at a specific location, that is, at a specific temperature, according to differential thermal analysis (DTA), which confirms that a specific chemical composition corresponds to the result of decomposition to analyze which component and how much is contained in the corresponding fire-damaged concrete structure sample. In addition, as illustrated by a black line in FIG. 10, it can be seen how the overall mass is reduced according to thermogravimetric analysis (TG).

In the case of the deep learning-based system 100 for time-dependent assessment of concrete deterioration according to the embodiment of the present invention, the result value of the black line (TG) in FIG. 10 is used, and since the concrete structure sample exposed to a specific fire damage temperature has already undergone mass reduction, it shows the feature that the mass loss does not occur in a certain section, and thus, it can be seen that the higher the fire damage temperature, the smaller the total mass reduction rate.

Therefore, by directly coring and taking the fire-damaged concrete structure sample from the fire site by the CPA application unit 140, and performing the TGA, the FTIR analysis and unclassified chemical analyses, the correlation with the high temperature exposure temperature may be accurately identified through the chemical and physical experiments.

Referring back to FIG. 8, the first DIA application unit 151 of the DIA application unit 150 uses a single analysis result of the TGA unit 141 as input data to derive a final output for calculating the fire damage temperature for damage depth inside a fire-damaged concrete structure and a fire temperature curve of a part corresponding to a heat source outside the concrete structure.

When a correlation value derived from the first DIA application unit 151 is below an appropriate level, the second DIA application unit 152 that uses the analysis result of the TGA unit 141, the analysis result of the FTIR analysis unit 142, and the analysis result of the chemical analysis unit 143 as input data to weight and re-analyze a clearly appearing feature, and derives the final output for calculating the fire damage temperature for each damage depth inside the concrete structure and the fire temperature curve of the part corresponding to the heat source outside the concrete structure. In this case, the feature is a result value that appears clearly at a specific temperature.

The data comparison and analysis unit 153 of the DIA application unit 150 compares the analysis result of the first DIA application unit 151 or the analysis result of the second DIA application unit 152 with the pre-constructed standardized experimental data.

In this case, as described above, the big data storage unit 160 stores other field investigation data, standardized experimental data, and history information, where the standardized experimental data includes a data storage space in which the physical and chemical behavior characteristics of a concrete sample are analyzed for each exposure temperature using various chemical analysis devices under strictly controlled laboratory conditions, and then the results are standardized and organized.

Specifically, as illustrated in FIG. 9, in the deep learning-based system for time-dependent assessment of concrete deterioration according to the embodiment of the present invention, the big data storage unit 160 stores other field investigation data, standardized experimental data, and history information, the deep learning-based system may expand and assess not only a sample collection location but also expand to and assess a member unit or an entire structure unit by synthesizing analysis results of concrete structure samples collected from specific locations at the site with statistical data such as other field investigation data, the standardized experimental data, the history information, and the like in the big data storage unit, in a process of applying the DIA, and increase a predictive rate by optimizing the assessed result.

Here, the standardized experimental data refers to a storage space that includes a TGA analysis result indicating a mass reduction rate for each exposure temperature of the concrete sample under standardized laboratory conditions, an FTIR analysis result indicating a change in chemical composition for each exposure temperature, and other various chemical analysis results, the other field investigation data refers to a storage space that includes information obtained through various interviews, CCTV analysis, and communication obtained through a corresponding fire site investigation, the history information refers to a storage space that includes information organized by converting a fire site analysis result generated in the meantime into data, and the big data storage unit 160 may comprehensively include all such information.

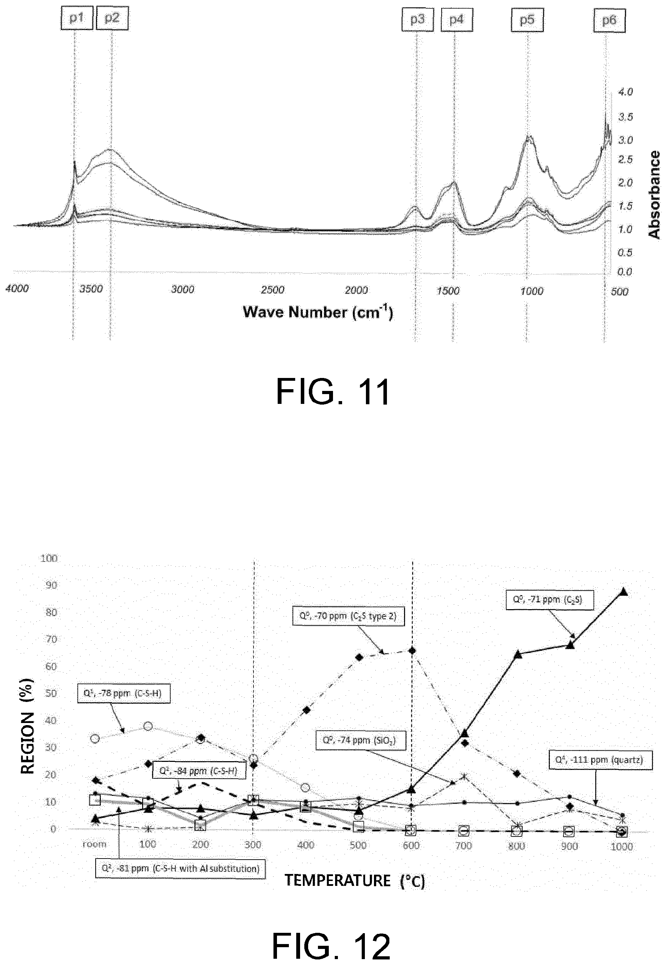

FIG. 11 is a diagram illustrating an FTIR analysis result for constructing the standardized experimental data of the big data storage unit in the deep learning-based system for time-dependent assessment of concrete deterioration according to the embodiment of the present invention.

As illustrated in FIG. 11, in the case of the deep learning-based system for time-dependent assessment of concrete deterioration according to the embodiment of the present invention, as the FTIR analysis result for constructing the standardized experimental data of the big data storage unit 160, depending on the fire damage temperature, a maximum value of a peak appearing at a specific wavelength number may vary or the area of the corresponding part may appear differently.

In the case of the deep learning-based system 100 for time-dependent assessment of concrete deterioration according to the embodiment of the present invention, the standardized experimental data may be constructed through the FTIR analysis.

Specifically, the FTIR analysis analyzes cement and blast furnace slag, respectively, and then, compares the analyzed cement and blast furnace slag. In the past, after defining a material that exhibits a peak at a specific wavelength number, it is possible to distinguish mixed substances according to the large and small values.

In the case of the deep learning-based system 100 for time-dependent assessment of concrete deterioration according to the embodiment of the present invention, as illustrated in FIG. 12, the maximum value of the peak appearing at the specific wavelength number according to the fire damage degree varies or the area of the corresponding part appears differently. In this case, it can be seen that the higher the fire damage temperature as a whole, the smaller the maximum value of the peak and the area of the part.

For example, in the case of the deep learning-based system 100 for time-dependent assessment of concrete deterioration according to the embodiment of the present invention, as illustrated in FIG. 12, after six peaks appearing at a specific wavelength number are determined, the values at that time are listed sequentially. In this case, one graph may be expressed with about 6 numbers and then collected to show the correlation with the temperature. For example, the main peak values of the sample exposed 600° are p1 = 1.54, p2 = 1.20, p3 = 1.02, p4 = 1.23, p5 = 1.64, and p6 = 1.52.

Therefore, by applying a deep neural network (DNN) model using a deep learning analysis tool by the DIA application unit 150, it is possible to increase the predictive rate of the fire damage degree for the fire-damaged concrete structure.

Referring back to FIG. 8, the fire damage temperature prediction unit 171a of the fire damage scale assessment unit 171 included in the deterioration assessment unit 170 predicts a fire damage temperature at a specific location for each damage depth of the fire-damaged concrete structure according to the deep learning interface algorithm application unit 150.

The fire temperature curve calculation unit 171b of the fire damage scale assessment unit 171 calculates a fire temperature curve according to a size of a flame corresponding to a first heat source outside the fire-damaged concrete structure according to the deep learning interface algorithm application unit 150. In this case, the fire temperature curve calculation unit 171b compares and analyzes the result value obtained from the outer surface of the concrete structure and the result value obtained from the inner region, which are each a piece of data analyzed in units of one set, and expresses the compared and analyzed result value as a function of time t to calculate the fire temperature curve according to the size of the flame corresponding to the first heat source outside the concrete structure, and the fire temperature curve may be given by the magnitude of the hydrothermal temperature over time and the value of the exposure time. For example, an X axis indicates the exposure time and a Y axis denotes the hydrothermal temperature.

The thermal diffusion path prediction unit 171c of the fire damage scale assessment unit 171 predicts the thermal diffusion path over time outside the fire-damaged concrete structure according to the predicted fire damage temperature and the calculated fire temperature curve. Accordingly, the thermal diffusion path prediction unit 171c may identify the thermal diffusion path over time in response to the prediction of the fire damage temperature at the time of the fire for an unknown fire-damaged concrete structure sample collected from the fire site.

The flame movement path tracking reproduction unit 171d of the fire damage scale assessment unit 171 tracks and reproduces the heat movement path from the location of the fire at the fire site as a function of time according to the predicted fire damage temperature and the calculated fire temperature curve.

FIG. 12 is a diagram illustrating an NMR measurement result in the deep learning-based system for time-dependent assessment of concrete deterioration according to the embodiment of the present invention.

As illustrated in FIG. 12, as an NMR measurement result in the deep learning-based system for time-dependent assessment of concrete deterioration according to the embodiment of the present invention, the area occupied by C-S-H is calculated to predict the strength development and a decrease in strength.

Specifically, in the deep learning-based system 100 for time-dependent assessment of concrete deterioration according to the embodiment of the present invention, a method of predicting a thermal diffusion path and tracking and reproducing a flame movement path is as follows.

The existing analysis method determines the heat source as an approximate empirical value, first sets the value, and then tracks the movement path of the flame. In this case, the predictive rate is less than 30% and there is no simulation analysis tool that may be applied in various ways to all fire sites. In addition, the system for predicting concrete thermal damage has not been reported in domestic or foreign related technologies. Meanwhile, the deep learning-based system 100 for time-dependent assessment of concrete deterioration according to the embodiment of the present invention may track the heat source with a high predictive rate by back calculation by analyzing the concrete that has only the temperature damage information at the fire site.

In the case of the deep learning-based system 100 for time-dependent assessment of concrete deterioration according to the embodiment of the present invention, since samples are collected directly from fire-damaged concrete structures of all fire sites, specific values present in the existing literature are not used. In addition, the simulation implementation does not proceed using external values, rather, it uses a back calculation method that starts from the inner sample and moves to the outside. For example, by removing various environmental factors as the process proceeds based on the result value in molecular and atomic units of cement paste, it is possible to increase the predictive rate.

In particular, instead of matching the standardized experimental data one by one, samples collected from the corresponding location by coring and cut are grouped into at least 4 pieces and matched in 1 set unit, and an output is derived by finding all the matching corresponding values or the most similar values.

In addition, the maximum temperature and exposure time may be calculated according to the size of the heat source outside the concrete structure corresponding to the source of such thermal damage, and thus, the fire temperature curve may be derived.

In addition, the above-described standardized experimental data also has result values stored in units of one set. In this case, since the fire temperature curve corresponding to the heat source is also stored, by matching the standardized experimental data stored in the big data storage unit 160 with the analysis result of the fire-damaged concrete structure sample collected from the fire site, it is possible to easily calculate the fire damage temperature curve.

The fire temperature curve calculation may be used to accurately predict the thermal diffusion path outside the fire-damaged concrete structure, and may dramatically increase the predictive rate of the final result by being used to track and reproduce the flame movement path.

As a result, in the deep learning-based system 100 for time-dependent assessment of concrete deterioration according to the embodiment of the present invention, the big data storage unit 160 stores 1) the TGA result showing the mass reduction rate by the exposure temperature of the fire-damaged concrete structure sample collected from the fire site by coring, 2) the FTIR analysis result showing the change in chemical composition by the exposure temperature of the fire-damaged concrete structure sample collected from the fire site by coring, and 3) other various chemical analysis results of the fire-damaged concrete structure sample collected from the fire site by coring.

Accordingly, in the deep learning-based system 100 for time-dependent assessment of concrete deterioration according to the embodiment of the present invention, after the physical and chemical analyses of the fire-damaged concrete structure sample collected from the fire site, by predicting the fire damage temperature for each damage depth of the fire-damaged concrete structure and calculating the fire temperature curve based on deep learning, it is possible to accurately predict the thermal diffusion path of the fire-damaged concrete structure and easily track and reproduce the flame movement path.

Meanwhile, the deep learning-based system 100 for time-dependent assessment of concrete deterioration according to the embodiment of the present invention is implemented by the FIS analysis tool, and each of the PSD unit 110, the ED unit 130, the CPA application unit 140, and the DIA application unit 150 will be described in detail as follows.

The PSD unit 110 may be applied to all of normal concrete structures, aged concrete structures, and structures damaged by disasters such as earthquakes and fires. For example, it may be applied to constituent members such as columns, beams, and slabs of concrete structures. In this case, coring (drilling) is performed using a drill at at least two locations in one constituent member.

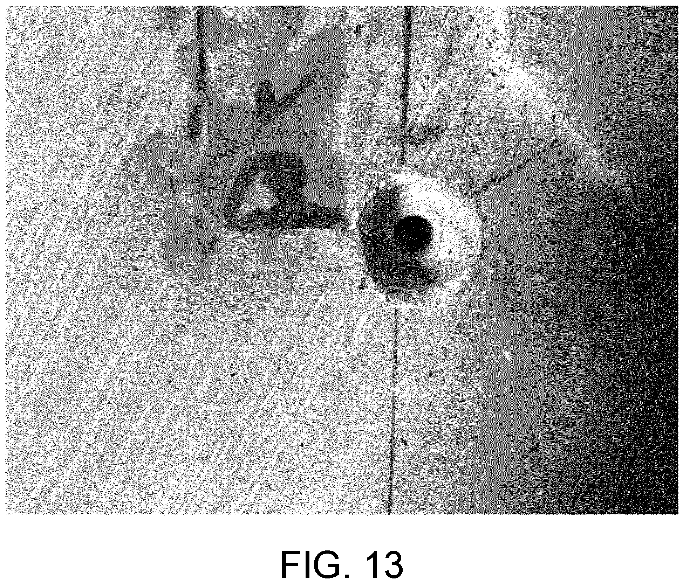

FIG. 13 is a diagram illustrating a surface of a concrete structure after sampling as a PSD process in the deep learning-based system for time-dependent assessment of concrete deterioration according to the embodiment of the present invention.

The concrete structure sample obtained through such sampling is additionally cut or processed into a concrete structure sample for analysis in an optimal state through crushing or other methods according to the requirements of the analysis device. Thereafter, as described below, the sample contamination source is calibrated by the sample contamination source calibration unit 190 for the collected concrete structure sample.

Next, the ED unit 130 is performed in parallel with various unclassified chemical analysis devices, such as NMR, TGA, FTIR, XRF, and XRD, depending on the purpose.

Next, as illustrated in FIG. 5 described above, the CPA application unit 140 performs the chemical analysis using at least one or more analysis devices, and modifies and codes the experimental results according to the CPA so that the experimental results reach a reliable level. In other words, only the traces remaining in the concrete structure sample are analyzed, and values modified by other external environmental factors are regarded as the sample contamination source, and the sample contamination sources are chemically separated and removed. In addition, it is possible to derive values necessary for prediction by synthesizing the result values obtained from at least one or more.

Next, as illustrated in FIG. 5 described above, the DIA application unit 150 measures residual stoichiometry at the corresponding location by comparing each analysis result of applying the DIA by the deep neural network with the pre-constructed standardized experimental data. In this case, the standardized experimental data may be stored in the big data storage unit 160, and the method of comparison and analysis with the standardized experimental data applies the DIA by the deep neural network.

In addition, each piece of data analyzed in units of one set, that is, the result value obtained from the surface of the concrete structure and the result value obtained from the inner region of the concrete structure are compared and analyzed and expressed as a function of time t, so the deterioration damage for each depth from the outer surface portion of the concrete structure to the location of the corresponding depth may be expressed as a function of time t, and also, residual performance such as residual life and residual strength may be assessed. For example, as illustrated in FIG. 12 described above, as the measurement result of the NMR, the area occupied by C-S-H may be calculated to predict strength development and a decrease in strength.

The deep learning-based system 100 for time-dependent assessment of concrete deterioration according to the embodiment of the present invention uses the FIS analysis tool to quantitatively analyze the residual chemical compositions inside the on-site concrete structure, so the level of concrete deterioration including the fire damage, the strength development, the carbonation damage scale, the salt damage scale, and the damage scale caused by unclassified chemical attacks may be scientifically assessed.

In addition, the deep learning-based system 100 for time-dependent assessment of concrete deterioration according to the embodiment of the present invention includes the big data storage unit 160, and may secure and store field investigation data, standardized experimental data, and the history analyzed in the past in the big data storage unit 160 to increase the predictive rate of the above-described process of applying the DIA, and may also include a wholistic approach process that expands a target to be analyzed to the entire concrete structure as well as sample locations in the concrete structure sampled at the site and an optimization process to obtain a result from areas where sampling was not performed during this wholistic approach process.

Meanwhile, referring back to FIG. 5, when the concrete structure sample is unintentionally contaminated due to the influence of the external environment during the process of collecting, processing, and analyzing the concrete structure sample, the sample contamination source calibration unit 190 calibrates the concrete structure sample by physically or chemically removing contaminants from the concrete structure sample so that the deterioration of the concrete structure may be reliably assessed. Here, the sample contamination source calibration unit 190 includes a sample contamination source physical calibration unit 191 and a sample contamination source chemical calibration unit 192.

For example, the main reasons for the contamination of the concrete structure sample collected from the site are the case where the collected concrete structure samples are directly exposed to the atmosphere for a long time and the case where a secondary chemical reaction occur inside the concrete structure samples due to strong external stimuli during the processing process, and the like.

To describe in more detail, when a concrete structure is damaged by a disaster such as a fire or earthquake, it is necessary to calibrate the concrete structure sample in consideration of the effect of the elapsed time from the time the damage occurred until the concrete structure sample was collected. In addition, even in the case of an aged concrete structure, it is necessary to calibrate the concrete structure sample in consideration of the effect of the collection process after the start of collecting the concrete structure sample and the time the concrete structure sample is directly exposed to the atmosphere after the sample is collected. In addition, in all processes of handling the concrete structure sample, it is necessary to calibrate the concrete structure sample by considering the effect of seasonal relative humidity and temperature. In addition, it is necessary to calibrate the concrete structure sample in consideration of the effect of external applied physical stimuli that may occur during the concrete structure sample processing process, for example, the cutting, crushing, and preparation process of concrete structures. In addition, it is necessary to calibrate the concrete structure sample in consideration of the effect occurring during the concrete structure sample analysis process.

As the calibration method by the sample contamination source calibration unit 190, first, there is a physical sample calibration method in which contaminants contaminating the concrete structure sample are physically removed using a centrifuge to calibrate the sample, or a chemical sample calibration method of calibrating the concrete structure sample when the CPA is applied.

First, in the physical sample calibration method performed by the sample contamination source physical calibration unit 191, after physically separating each constituent material of the concrete structure samples collected from the site using the centrifuge, the main experiment is conducted only for the target concrete structure sample to derive the final result.

Next, the sample contamination source chemical calibration unit (192) cross-confirms whether the contamination source is included using one or more chemical analysis devices, and then removes the contamination source when the contamination source is included to derive a calibrated value as the final value or selects only the results of some sections or specific chemical compositions in the chemical analysis result using a single device to be derived as the final result. For example, the results obtained from at least two or more chemical analysis devices are comprehensively analyzed and removed, and in this case, each chemical analysis result obtained using different devices is comprehensively analyzed to remove the region recognized as a contaminant and then is derived as the final result value.

To describe in more detail, the method of physically removing contaminants included in a concrete structure sample using a centrifuge is as follows

The method using a centrifuge is applied when fine substances that are difficult to separate by sieving are mixed. For example, the fine substances may correspond to dust generated from concrete aggregate. In this case, a concrete structure sample mixed with two or more constituent materials such as cement, an aggregate, and an admixture is put into a centrifuge, and after setting a rotation speed, time, and temperature, it is operated to separate various types of samples independently.

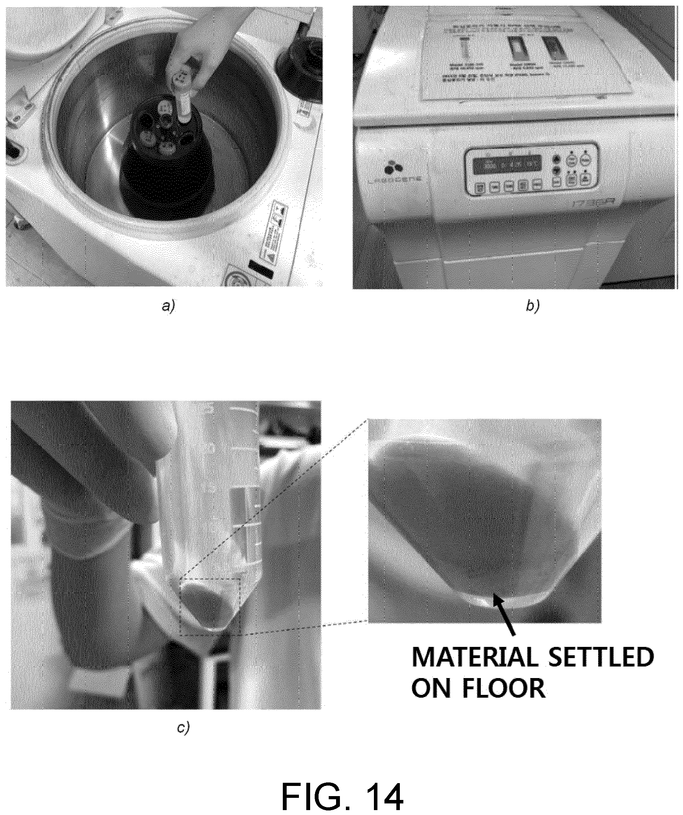

FIG. 14 is a photograph showing the physical removal of contaminants by centrifugation as a sample contamination source physical calibration in the deep learning-based system for time-dependent assessment of concrete deterioration according to the embodiment of the present invention.

FIG. 14A illustrates that a sample is inserted into a sample holder and prepared for the test, FIG. 14B illustrates that centrifugation is started, and FIG. 14C illustrates that the centrifugation is confirmed and substances settled on the bottom are confirmed.

There is a method of calibrating a concrete structure sample when applying a CPA.

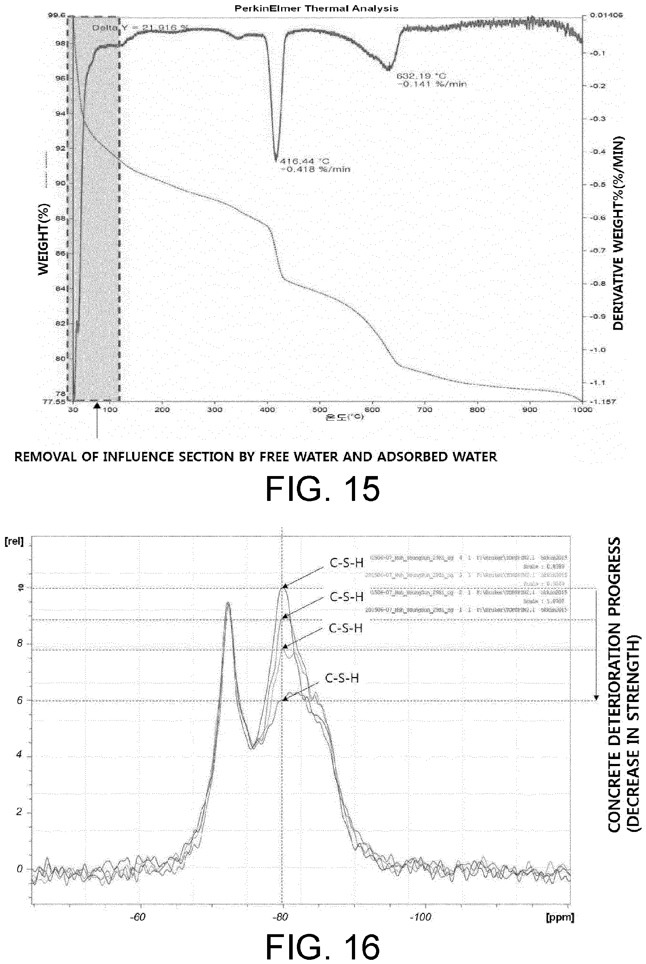

FIG. 15 is a diagram illustrating the removal of a contaminated section after TGA as sample contamination source chemical calibration in the deep learning-based system for time-dependent assessment of concrete deterioration according to the embodiment of the present invention.

As illustrated in FIG. 15, the mass reduction amount generated in this region may be removed from the cumulative mass reduction rate calculation and then derived as the final result value.

In addition, as another method, in the case of NMR, after putting the collected sample in a probe tube and inserting the probe tube into the center of a superconductor, by performing high-speed spinning in a constant magnetic field state, and then applying magnetic pulses at regular intervals to generate resonance, the unique resonance frequency for each sample may be calculated as a graph by applying the Fourier transform. In this way, a chemical bonding state of silicon (Si) present in the cement paste may be measured. For example, when a cement paste sample is analyzed through NMR 29Si, the chemical bond states such as C3S, C2S, SiO₂, C-S-H, and C-A-H may be confirmed.

Among them, by selecting a region where the state of C-S-H that directly affects the strength of concrete may be confirmed and observing the size of the peak, the level of deterioration and strength development of concrete may be derived as the final value.

FIG. 16 is a diagram illustrating the selection and analysis of a specific section in which C-S-H is present after NMR Si analysis as sample contamination source chemical calibration in the deep learning-based system for time-dependent assessment of concrete deterioration according to the embodiment of the present invention.

For example, as illustrated in FIG. 16, after NMR 29Si analysis, the presence of C-S-H in the -78 ppm section may be selected and analyzed.

In general, concrete is composed of various elements such as Ca, Si, Al, Fe, S, Mg, Ti, Sr, P, Mn, Zn, and K. The values of the elements change according to the amount occupied by cement paste and aggregates, which are concrete constituent materials.

When this is quantitatively analyzed, the contamination source of the concrete structure sample may be identified and removed.

Accordingly, according to an embodiment of the present invention, in relation to the sampling method in the on-site reinforced concrete structure, according to the related art, a tremendous amount of time and effort is required to collect a coring sample with a size of 100 × 200 mm from the concrete structure, in particular, the sampling device for collecting concrete structure samples should be necessarily fixed with anchor bolts, and the location of the rebar should be necessarily searched for.

On the other hand, according to the embodiment of the present invention, since a 10 × 40 mm drill is used, there is no need to fix with an anchor bolt, and, for example, sampling may be performed with a mobile hand drill or a hand core device. In this case, there is no need to search for rebar positions.

In addition, in relation to collecting a fine powder sample from an on-site reinforced concrete structure, according to the related art, a sample having a size of 100 × 200 mm is collected from the site by coring, transported to a laboratory, and sampled using an expensive concrete fine powder collector, or when there is no collecting device, additional efforts such as cutting and crushing the concrete structure sample are required. In particular, it is necessary to transport the concrete structure samples from the site to the laboratory, and when dry collection is required, it is more difficult to collect the concrete structure sample.

On the other hand, according to an embodiment of the present invention, the concrete structure sample may be taken immediately using a drill bit at the site. In this case, since immediate sampling may be performed at the site, there is no need to transport the concrete structure sample to the laboratory and 100% dry collection is possible.

In addition, it is necessary to analyze the characteristics of one desired concrete constituent material by physically and chemically separating only cement paste or aggregate from the on-site reinforced concrete structure. According to the embodiment of the present invention, by using the centrifugation method using the difference in density of the concrete constituent materials or by applying the CPA, it is possible to separate the ratio of cement paste and aggregate from the concrete structure sample with high accuracy.

In addition, in relation to the fire damage temperature prediction using a TGA analysis device, according to the related art, the TGA device was not used as a fire damage temperature prediction device because it was inconsistent according to temperature change, but according to an embodiment of the present invention, by deriving the final result by selectively removing the result value near the 100°C region corresponding to the contamination source from the TGA analysis result, it is possible to simply use the TGA device as the fire damage temperature prediction device.

In addition, in relation to the prediction of a decrease in strength of the on-site reinforced concrete structure using an NMR device, according to an embodiment of the present invention, since only cement paste may purely be separated from the on-site reinforced concrete structure, it is possible to select and analyze only C-S-H corresponding to a specific chemical composition using the NMR device, and thus, it is possible to easily analyze the strength development and decrease of the concrete structure.

In addition, in relation to predicting the deterioration of the on-site reinforced concrete structures using unclassified chemical analysis device, according to the related art, since there is no original technology that may completely separate cement paste and aggregate from concrete, the chemical analysis itself has not been attempted. In some cases, data analyzing concrete structure samples have been reported, but since the ratio of the amount of aggregate is different for each, there has been no report of consistent analysis results. Therefore, there is no field application example. In this case, the neutralization field measurement method is difficult to view as chemical analysis and is a simple way to check whether there is reaction with the phenolphthalein solution. For reference, aggregate fine powder is not visually distinguished from cement paste.

On the other hand, according to an embodiment of the present invention, when using the method of purely separating cement paste from the on-site reinforced concrete structure by applying the above-described CPA, in the meantime, various chemical analysis methods obtained in the laboratory through literature data can be applied to the site, so it is possible to analyze the deterioration state of concrete structures at the site in a more extensive and diverse way.

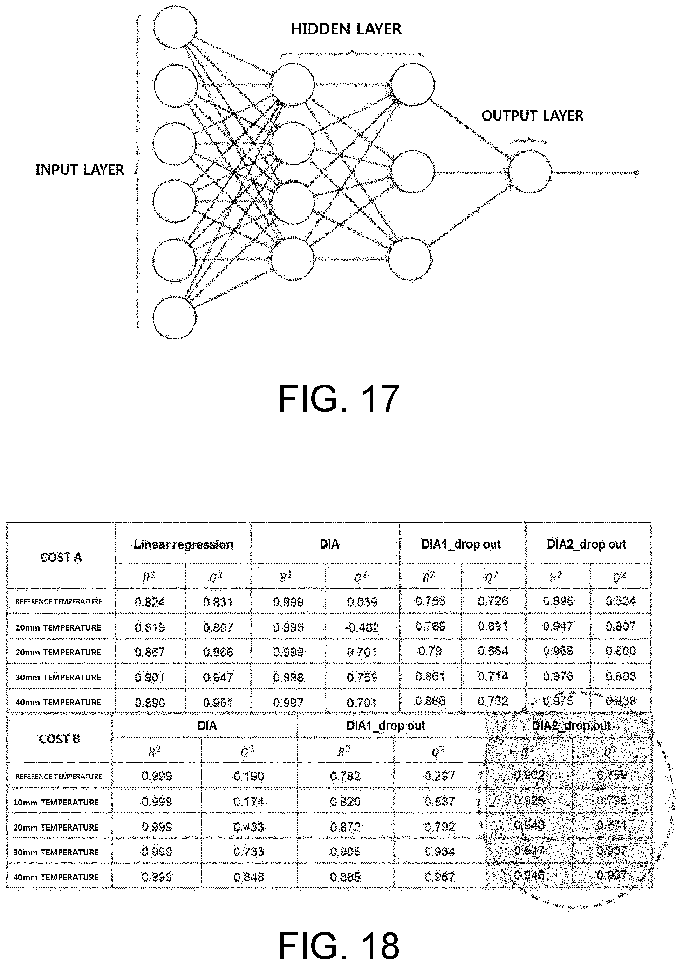

Meanwhile, FIG. 17 is a diagram schematically illustrating a deep learning interface algorithm (DIA) applied to the deep learning-based system for time-dependent assessment of concrete deterioration according to the embodiment of the present invention, and FIG. 18 is a diagram illustrating the selection of a DIA with high predictive power by searching for a DIA applicable to the deep learning-based system for time-dependent assessment of concrete deterioration according to the embodiment of the present invention.

In the case of the deep learning-based system 100 for time-dependent assessment of concrete deterioration according to the embodiment of the present invention, a DIA is applied, and the process of applying the DIA will be described in detail as follows.

First, as illustrated in FIG. 17, the DIA is an artificial neural network (ANN) including multiple hidden layers between an input layer and an output layer. The DIA corresponds to one of machine learning methods and by including multiple hidden layers, various nonlinear relationships can be learned.

In general, such DIA analysis derives a function formula that predicts a certain characteristic based on training data. For example, a k value may be derived from Hooke's law F = kx.

The standardized experimental data described above was constructed by selecting only predetermined features from the TGA and FTIR analysis results only for DIA analysis. In other words, the standardized experimental data is data that is digitized and organized after selecting a specific section or specific value as a representative value. For example, only specific values are extracted and organized from the standardized experimental data.

In the case of the deep learning-based system 100 for time-dependent assessment of concrete deterioration according to the embodiment of the present invention, after searching for an applicable DIA and analyzing the results, by performing a process of selecting and optimizing a DIA with high predictive power, the optimized DIA is completed.

The completed DIA model is implemented in the form of software, and as illustrated by the broken line in FIG. 18, the most similar value is found by matching the concrete structure sample collected from the site with the pre-constructed standardized experimental data sample.

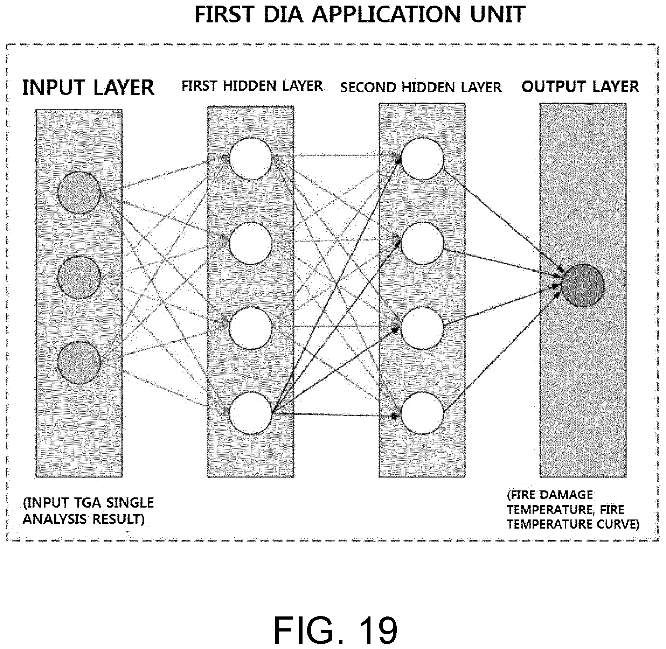

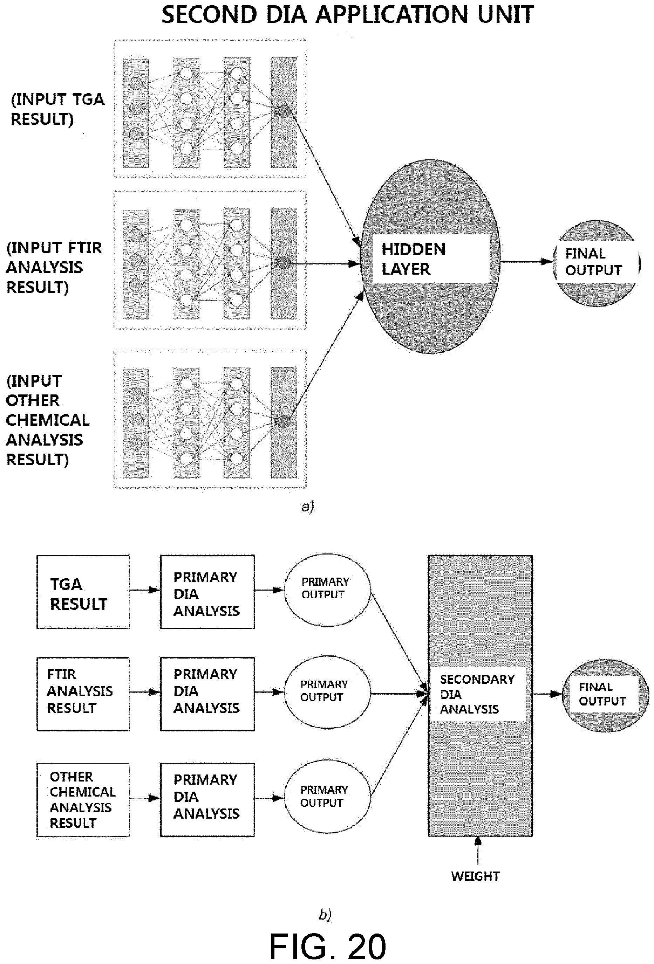

Meanwhile, FIG. 19 is a diagram illustrating a first DIA application unit in the deep learning-based system for time-dependent assessment of concrete deterioration according to the embodiment of the present invention, and FIG. 20 is a diagram illustrating a second DIA application unit in the deep learning-based system for time-dependent assessment of concrete deterioration according to the embodiment of the present invention.

More specifically, in the deep learning-based system 100 for time-dependent assessment of concrete deterioration according to the embodiment of the present invention, the DIA proceeds by mixing two methods, the first DIA application unit and the second DIA application unit, but when the result value of applying the first DIA is equal to or greater than the appropriate level, the analysis is terminated. However, when the correlation value is less than an appropriate level, it is possible to improve the accuracy of the final result value by additionally applying the second DIA.

First, in the case of the first DIA application unit, as illustrated in FIG. 19, for example, a TGA result alone is used as input data, and finally, the fire damage temperature inside the concrete structure, which is the result, and the fire temperature curve corresponding to the heat source outside the fire-damaged concrete structure are calculated respectively.

In addition, when the correlation of the first DIA application result is low, re-analysis is performed. In the case of the second DIA application result, as illustrated in FIG. 20A, it corresponds to the method of performing re-analysis by weighting clearly appearing features of the TGA result, the FTIR analysis result, and other various chemical analysis result values. In other words, analysis is performed by weighting the result values that appear clearly at a specific temperature.

In addition, as illustrated in FIG. 20B, a weight is assigned to result values having a high predictive rate in a specific temperature range for each analysis device. For example, since the predictive rate of the FTIR analysis result value at a temperature of 100°C or lower is high, the FTIR analysis result value is assigned a weight at 100°C or lower, and the TGA result value is assigned a weight at 100°C or higher, and after a comprehensive analysis, the final output may be derived.

In this case, as the result of applying the first DIA described above, the output is calculated by calculating each of the fire damage temperature for each depth inside the concrete structure and the fire temperature curve of the part corresponding to the external heat source of the concrete structure, so it is possible to greatly increase the predictive rate of the thermal diffusion path and the flame movement path.

The result values of the first DIA application unit and the second DIA application unit are the fire damage temperature inside the fire-damaged concrete structure. That is, after the fire-damaged concrete structure sample cored at the fire site is analyzed, when the analyzed fire-damaged concrete structure sample is input,as input data, to the software to which the DIA is applied and then passes through a matrix to which the weight is assigned by the matching algorithm, finally, it is possible to predict the fire damage temperature at the cored fire-damaged concrete structure sample location and to calculate the fire temperature curve outside the fire-damaged concrete structure. This may be used for damage scale and damage degree assessment of the fire-damaged concrete structure, for suggesting the recovery plan, and the like.

As a result, according to an embodiment of the present invention, the fire damage scale assessment, most concrete structure deterioration may be assessed through deep learning-based chemical analysis, such as the strength development assessment, the carbonation damage scale assessment, the salt damage scale assessment, and unclassified chemical attack damage scale assessment of the concrete structure using the FIS analysis tool for concrete structures.

In addition, according to an embodiment of the present invention, in the process of applying the DIA of the FIS analysis tool for concrete structures, by synthesizing statistical data such as other field investigation data, the standardized experimental data, and the history information in the big data storage unit and the concrete structure sample analysis results collected from specific locations at the site, it is possible to expand and assess not only the sampling location but also expand to and assess the member unit or the entire structure unit, and optimize the assessed result to increase the predictive rate.

In addition, according to an embodiment of the present invention, to increase the accuracy of the time-dependent assessment of concrete deterioration results, physical calibration and chemical calibration are performed on the contamination source of the concrete structure sample, and the deterioration damage scale of the concrete structure is clearly assessed according the depth change, so it is possible to correctly diagnose the deterioration of the concrete structure..

### [Deep Learning-Based Method of Time-Dependent Assessment of Concrete Deterioration]

FIG. 21 is an operation flowchart of a deep learning-based method of time-dependent assessment of concrete deterioration according to an embodiment of the present invention.

Referring to FIG. 21, in the deep learning-based method of time-dependent assessment of concrete deterioration according to an embodiment of the present invention, first, as a PSD process, practical sampling is performed on a concrete structure to collect a concrete structure sample from the site, process the collected concrete structure sample into a sample for analysis in an optimal state, and store the sample (S 110). For example, the concrete structure sample is collected by drilling or coring the building members of a wall, a column, or a slab of a concrete structure, in which the drilling or coring may be performed up to a location of the main rebar with a minimum diameter and a depth of at least 40 mm. In addition, the concrete structure sample collected by drilling or coring is defined, as one set of at least two or more concrete structure samples at one location, and all analyses are performed in units of one set, and an output is derived by finding all the corresponding values matching or the values most similar to the pre-constructed standardized experimental data. In addition, one set unit analysis may show a thermal diffusion rate as a function of time when comparing the result values for each section by analyzing at least two samples taken from one location through the drilling or coring.

Next, the primary constituent materials of the concrete structure sample are physically separated and processed (S120).

Next, as the ED process, chemical analysis is performed on the processed concrete structure sample using at least one chemical analysis device (S130). In this case, the chemical analysis may be performed on the concrete structure sample collected and processed by drilling or coring in the field using at least one of XRD, XRF, FTIR, NMR, or TGA.

Next, as the process of applying a CPA, the secondary constituent materials of the concrete structure sample are chemically separated and the chemical analysis result is coded (S140). For example, when the deterioration assessment of the concrete structure sample is the fire damage scale assessment, the TGA unit 141 may perform TGA measuring the mass reduction rate of the concrete structure sample, and the FITR analysis unit 142 may analyze the maximum value of the peak appearing at a specific wavelength number and the area of the corresponding part according to the fire damage temperature of the concrete structure sample, and the chemical analysis unit 143 may perform the chemical analysis in parallel with chemical analysis device according to the purpose of analysis.

Next, as the process of applying a DIA, the DIA is applied to the chemical analysis result for deep learning analysis and compared with the pre-constructed standardized experimental data to derive the optimal analysis results (S150). For example, a first DIA is applied to derive the final output for calculating the fire damage temperature for each damage depth inside the fire-damaged concrete structure and the fire temperature curve of the part corresponding to the external heat source of the fire-damaged concrete structure by using the single analysis result of the TGA unit 141 as input data, and when the correlation value of the first DIA is below an appropriate level, the analysis result of the TGA unit 141, the analysis result of the FTIR analysis unit 142, and the analysis result of the chemical analysis unit 143 are used as input data to weight and re-analyze clearly appearing features, and a second DIA is applied to derive the final output for calculating the fire damage temperature for each damage depth inside the concrete structure and the part corresponding to the heat source outside the concrete structure. In addition, the analysis result of the first DIA or the analysis result of the second DIA is compared with the pre-constructed standardized experimental data.

Next, the deterioration of the concrete structure sample is assessed (S160). In this case, the fire damage scale, strength development, carbonation damage scale, salt damage scale, and unclassified chemical attack damage scale are assessed for the concrete structure sample. For example, in the case of the fire damage scale assessment for the concrete structure sample, the result value obtained from the outer surface of the fire-damaged concrete structure, which is each piece of data analyzed in units in one set, and the result value obtained from the inner region are compared and analyzed and are expressed as a function of time t, and by comparing and analyzing the results of the fire-damaged concrete structure sample cored in the periphery, the fire temperature curve is calculated according to the size of the flames corresponding to the first heat source outside the fire-damaged concrete structure, but the fire temperature curve may be given as the magnitude of the hydrothermal temperature over time and the value of the exposure time.

Next, a simulation is implemented to predict the deterioration progress rate of the concrete structure as a function of time t according to the deterioration assessment result to derive key information, and the deterioration damage scale by cause is assessed and coded (S170).

Next, the deterioration assessment result of the concrete structure sample is stored in the big data storage unit 160 (S 180). Here, the big data storage unit 160 stores other field investigation data, the standardized experimental data, and the history, and the standardized experimental data may include a TGA result showing the mass reduction rate for each exposure temperature of the concrete structure sample collected from the site by coring, an FTIR analysis result showing the change in chemical composition for each exposure temperature of the concrete structure sample collected from the site by coring, and other various chemical analysis results of the concrete structure sample collected from the site by coring.

Meanwhile, the deep learning-based method of time-dependent assessment of concrete deterioration according to the embodiment of the present invention further include removing contaminants contaminating the concrete structure sample or calibrating the contamination cause so that the analysis result of the concrete structure sample is not affected. In this case, when the concrete structure sample is processed in operation S120, the contamination source of the concrete structure sample is physically removed to calibrate the concrete structure sample. In addition, when the CPA is applied in operation S 140, the concrete structure sample may be calibrated by chemically removing the contamination source of the concrete structure sample.

For example, when physically removing the contaminants of the concrete structure sample, the contaminants contaminating the concrete structure sample are physically removed using a centrifuge to calibrate the concrete structure sample, but after physically separating each constituent material of the concrete structure sample collected from the site using a centrifuge, the final result may be derived by performing this experiment only on the target concrete structure sample.

In addition, when chemically removing the contamination source of the concrete structure sample, after it is cross-confirmed whether the contamination source is included using one or more chemical analysis devices, when the contamination source is included, the contamination source is removed to derive a calibrated value as the final value or only the results of some sections or specific chemical compositions may be selected from the chemical analysis results using a single device to be derived as the final result.

As a result, according to the present invention, it is possible to predict the deterioration progress rate of a concrete structure as a function of time, and in particular, it is possible to implement a simulation by expanding to member units and entire structure units, secure the highest predictive rate compared to the existing method, and achieve the fastest analysis. In addition, according to the present invention, it is possible to simultaneously assess most deterioration levels related to a concrete structure, and monitor post-quality of the concrete structure from immediately after completion to demolition.

The above description of the present invention is for illustrative purposes, and those skilled in the art to which the present invention pertains will understand that it may be easily modified into other specific forms without changing the technical spirit or essential features of the present invention. Therefore, it should be understood that the above-mentioned embodiments are exemplary in all aspects and not limiting. For example, each component described as a single type may be implemented in a distributed manner, and similarly, components described as distributed may be implemented in a combined form.

It is to be understood that the scope of the present invention is defined by the claims rather than the above-described description and all modifications and alternations derived from the claims and their equivalents are included in the scope of the present invention.

## Claims

1. A deep learning-based system for time-dependent assessment of concrete deterioration, comprising:
a practical sampling design (PSD) unit (110) that performs practical sampling on a concrete structure to determine a sampling location and quantity from an on-site concrete structure, collect a sample, process the collected sample into an optimal sample for analysis, and store the optimal sample for analysis
a sample processing unit (120) that physically separates and processes primary constituent materials of the concrete structure sample;
an experimental design (ED) unit (130) that performs chemical analysis on the concrete structure sample processed in the sample processing unit (120) using at least one chemical analysis device;
a chemical profiling algorithm (CPA) application unit (140) that chemically separates a secondary constituent material of the sample of the concrete structure and applies CPA for coding a chemical analysis result;
a deep learning interface algorithm (DIA) application unit (150) that applies a DIA to an analysis result of the ED unit (130) to compare with pre-constructed standardized experimental data and derive an optimal analysis result; and
a deterioration assessment unit (170) that assesses a deterioration damage scale of the sample of the concrete structure, and includes fire damage scale assessment, strength development assessment, carbonation damage scale assessment, salt damage scale assessment, and unclassified chemical attack damage scale assessment for the sample of the concrete structure.

2. The deep learning-based system of claim 1, further comprising a forensic investigation of a concrete structure (FIS) analysis tool configured to scientifically quantitatively assess a deterioration damage scale from the collection of the sample of the concrete structure in association with the PSD unit (110), the ED unit (130), the CPA application unit (140), and the DIA application unit (150).

3. The deep learning-based system of claim 1, further comprising a time dependent assessment (TDA) derivation unit (180) that implements simulation to predict a deterioration progress rate of the concrete structure according to the deterioration assessment result of the deterioration assessment unit (170) as a function of time t to derive key information, assess a deterioration damage scale by cause, and code the deterioration damage scale.

4. The deep learning-based system of claim 1, wherein the ED unit (130) performs the chemical analysis using at least one of X-ray diffraction (XRD), X-ray fluorescence (XRF), Fourier transform infrared (FTIR), nuclear magnetic resonance (NMR), or thermometric analysis (TGA).

5. The deep learning-based system of claim 1, wherein the CPA application unit (140) includes:
a TGA unit (141) that performs TGA measuring a mass reduction rate of the concrete structure sample;
an FTIR analysis unit (142) that analyzes a maximum value of a peak appearing at a specific wavelength number and an area of a corresponding part according to a fire damage temperature of the concrete structure sample; and
a chemical analysis unit (143) that performs chemical analysis using various chemical analysis devices in parallel according to the purpose of analysis.

6. The deep learning-based system of claim 5, wherein the higher the fire damage temperature in the TGA unit (141), the smaller the mass loss pattern and the total mass reduction rate appear, and the higher the overall fire damage temperature in the Fourier transform infrared analysis unit (142), the smaller the maximum value of the peak and the area of the part.

7. The deep learning-based system of claim 5, wherein the DIA application unit (150) includes:
a first DIA application unit (151) that uses a single analysis result of the TGA unit (141) as input data to derive a final output for calculating a fire damage temperature for each damage depth inside a fire-damaged concrete structure and a fire temperature curve of a part corresponding to a heat source outside the concrete structure;
a second DIA application unit (152) that, when a correlation value derived from the first DIA application unit (151) is below an appropriate level, uses an analysis result of the TGA unit (141), an analysis result of the FTIR analysis unit (142), and an analysis result of the chemical analysis unit (143) as input data to weight and re-analyze a clearly appearing feature, and derives a final output for calculating the fire damage temperature for each damage depth inside the concrete structure and the fire temperature curve of the part corresponding to the heat source outside the concrete structure; and
a data comparison and analysis unit (153) that compares an analysis result of the first DIA application unit (151) or an analysis result of the second DIA application unit (152) with the pre-constructed standardized experimental data,
wherein the feature of the second DIA application unit (152) is a clearly appearing result value at a specific temperature.

8. The deep learning-based system of claim 1, wherein the DIA application unit (150) compares and matches the analyzed data of the sample of the concrete structure collected from a site with pre-constructed statistical data, and applies a wholistic approach process that expands a target to be analyzed to the entire concrete structure as well as a sample location of the sample of the concrete structured at the site and an optimization process to obtain a result at a location where sampling was not performed during the wholistic approach process.

9. The deep learning-based system of claim 1, further comprising a big data storage unit (160) that stores other field investigation data, standardized experimental data, and history information,
wherein the standardized experimental data refers to a storage space that includes a TGA result indicating a mass reduction rate for each exposure temperature of the concrete sample under standardized laboratory conditions, an FTIR analysis result indicating a change in chemical composition for each exposure temperature, and other various chemical analysis results, the other field investigation data refers to a storage space that includes information obtained through various interviews, CCTV analysis, and communication obtained through a corresponding fire site investigation, the history information refers to a storage space that includes information organized by converting a fire site analysis result generated in the meantime into data, and the big data storage unit comprehensively includes all such information.

10. The deep learning-based system of claim 1, further comprising a sample contamination source calibration unit (190) that removes a contaminant contaminating the sample of the concrete structure or calibrates a contamination cause in order to increase accuracy of an analysis result of the sample of the concrete structure.

11. The deep learning-based system of claim 10, wherein the sample contamination source calibration unit (190) includes:
a sample contamination source physical calibration unit (191) that calibrates the sample of the concrete structure by physically removing a contamination source of the sample of the concrete structure when the sample processing unit (120) processes the sample of the concrete structure; and
a sample contamination source chemical calibration unit (192) that calibrates the sample of the concrete structure by chemically removing the contamination source of the sample of the concrete structure when the CPA of the CPA application unit (140) is applied.

12. The deep learning-based system of claim 11, wherein the sample contamination source physical calibration unit (191) calibrates the sample of the concrete structure by physically removing the contaminant contaminating the sample of the concrete structure using a centrifuge, and physically separates each constituent material of the sample of the concrete structure collected from a site using the centrifuge and then performs experiment only on the targeted concrete structure sample to derive a final result.

13. The deep learning-based system of claim 11, wherein the sample contamination source chemical calibration unit (192) cross-confirms whether the contamination source is included using one or more chemical analysis devices, and then, when the contamination source is included, removes the contamination source to derive a calibrated value as the final value or selects only results of some sections or specific chemical compositions from a chemical analysis result using single device to be derived as the final result.

14. The deep learning-based system of claim 1, wherein the deterioration assessment unit (170) includes:
a fire damage scale assessment unit (171) that predicts fire damage temperature for each depth of the concrete structure and predicts a fire damage temperature curve of a surface of the concrete structure;
a strength development assessment unit (172) that assesses a strength development level according to hydration delay and chemical damage for each depth of the concrete structure;
a carbonation damage scale assessment unit (173) that investigates a carbonation damage scale for each depth of the concrete structure and predicts a carbonation damage rate;
a salt damage scale assessment unit (174) that investigates a chloride damage scale for each depth of the concrete structure and predicts a salt damage rate; and
an unclassified chemical attack damage scale assessment unit (175) that investigates damage caused by corrosive gas and damage caused by salts of sulfate and acid for each depth of the concrete structure and predicts an unclassified chemical damage deterioration rate.

15. The deep learning-based system of claim 14, wherein the fire damage scale assessment unit (171) includes:
a fire damage temperature prediction unit (171a) that predicts fire damage temperature at a specific location for each damage depth of a fire-damaged concrete structure according to the DIA application unit (150);
a fire temperature curve calculation unit (171b) that calculates a fire temperature curve corresponding to a first heat source outside the fire-damaged concrete structure according to the DIA application unit (150);
a thermal diffusion path prediction unit (171c) that predicts a thermal diffusion path inside the fire-damaged concrete structure according to the predicted fire damage temperature and the calculated fire temperature curve; and
a flame movement path tracking reproduction unit (171d) that tracks and reproduces a heat movement path from a fire location at a fire site as a function of time according to the predicted fire damage temperature and the calculated fire temperature curve,
wherein the thermal diffusion path prediction unit (171c) identifies the thermal diffusion path over time in response to the prediction of the fire damage temperature at the time of the fire for an unknown fire-damaged concrete structure sample collected from the fire site.

16. The deep learning-based system of claim 15, wherein the fire temperature curve calculation unit (171b) collects samples for each depth from the same location, compares and analyzes each piece of data analyzed in units of one set, and expresses the data as a function of time t to calculate the fire temperature curve of the surface corresponding to a first heat source of the concrete structure, and the fire temperature curve is given by a magnitude of hydrothermal temperature over time and a value of the exposure time.

17. The deep learning-based system of claim 1, wherein the sample of the concrete structure is collected by drilling or coring a building member of a wall, a column, or a slab of a fire-damaged concrete structure, in which the drilling or coring is performed up to a location of a main rebar with a minimum diameter and a depth of at least 40 mm,
for the sample of the concrete structure collected by the drilling or coring, at least two or more concrete structure samples are defined as one set at one location, and all analyses are performed in units of one set, and an output is derived by finding all corresponding values matching or values most similar to the pre-constructed standardized experimental data, and
the one set unit analysis expresses a thermal diffusion rate as a function of time when comparing the result values for each section by analyzing at least two samples collected from one location through the drilling or coring.

18. A deep learning-based method of time-dependent assessment of concrete deterioration, comprising:
a) as a practical sampling design (PSD) process, performing practical sampling on a concrete structure to collect a sample of a concrete structure from a site, process the sample of the concrete structure into a sample for analysis in an optimal state, and store the processed concrete structure sample;
b) physically separating and processing a primary constituent material of the sample of the concrete structure;
c) as an experimental design (ED) process, performing chemical analysis on the processed concrete structure sample using at least one chemical analysis device;
d) as a process of applying a chemical profiling algorithm (CPA), chemically separating a secondary constituent material of the sample of the concrete structure and coding a chemical analysis result;
e) as a process of applying a deep learning interface algorithm (DIA), after applying DIA to the chemical analysis result for deep learning analysis, deriving an optimal analysis result by comparing with pre-constructed standardized experimental data;
f) assessing a deterioration of the sample of the concrete structure; and
g) implementing simulation to predict the deterioration progress rate of the concrete structure as a function of time t according to the deterioration assessment result, deriving key information, assessing and coding a deterioration damage scale by each cause,
wherein in operation f), fire damage, strength development, a carbonation damage scale, a salt damage scale, and an unclassified chemical attack damage are assessed for the sample of the concrete structure.

19. The deep learning-based method of claim 18, further comprising h) storing a deterioration assessment result of the sample of the concrete structure in a big data storage unit (160),
wherein the big data storage unit (160) stores other field investigation data, standardized experimental data, and history, the standardized experimental data refers to a storage space that includes a TGA result showing a mass reduction rate for each exposure temperature of a concrete sample under standardized laboratory conditions, an FTIR analysis result showing a change in chemical composition for each exposure temperature, and other various chemical analysis results, the other field investigation data refers to a storage space that includes information obtained through various interviews, CCTV analysis, and communication obtained through the corresponding fire site investigation, the history information refers to a storage space that includes information organized by converting a fire site analysis result generated in the meantime into data, and the big data storage unit comprehensively includes all such information.
